(19) Europäisches Patentamt
European Patent Office
Office européen des brevets

(11) EP 2 172 504 B1

(12) # EUROPÄISCHE PATENTSCHRIFT

(45) Veröffentlichungstag und Bekanntmachung des Hinweises auf die Patenterteilung:
**20.07.2011 Patentblatt 2011/29**

(51) Int Cl.:
***C08G 18/67*** (2006.01) ***C08G 18/77*** (2006.01)
***G03F 7/00*** (2006.01) ***G03F 7/027*** (2006.01)
***G11B 7/245*** (2006.01)

(21) Anmeldenummer: **09011898.5**

(22) Anmeldetag: **18.09.2009**

(54) **Photopolymerformulierungen mit niedriger Vernetzungsdichte**

Photopolymer formulations with low interlacing density

Formules de photopolymères ayant une densité de réseau réduite

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **01.10.2008 EP 08017276**

(43) Veröffentlichungstag der Anmeldung:
**07.04.2010 Patentblatt 2010/14**

(73) Patentinhaber: **Bayer MaterialScience AG**
**51368 Leverkusen (DE)**

(72) Erfinder:
- **Bruder, Friedrich-Karl, Dr.**
  **47802 Krefeld (DE)**
- **Weiser, Marc-Stephan, Dr.**
  **51379 Leverkusen (DE)**
- **Roelle, Thomas, Dr.**
  **51381 Leverkusen (DE)**
- **Fäcke, Thomas, Dr.**
  **51375 Leverkusen (DE)**
- **Hönel, Dennis**
  **53909 Zülpich (DE)**

(56) Entgegenhaltungen:
**EP-A- 0 945 762**
**US-A1- 2007 072 124**
**US-B2- 6 780 546**

EP 2 172 504 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Photopolymerformulierungen basierend auf einem polymeren Netzwerk als Matrix und mindestens einem darin gelösten photopolymerisierbaren Monomer sowie ein Verfahren zur Herstellung von holographischen Medien aus solchen Photopolymeren und deren Verwendung. Dabei weißt die Photopolymerformulierung vor der Belichtung als Maß für die Vernetzungsdichte ein besonderes mittleres Molekulargewicht $M_C$ der zwei Polymerstränge verbrückenden Segmente auf, bzw. ein besonderes Verhältnis Q dieser Vernetzungsdichte zur Molmasse $M_{Mo}$ des gelösten Schreibmonomeren auf, ausgedrückt als $Q = M_C / M_{Mo}$.

Photopolymere stellen Materialien dar, die mittels der Überlagerung zweier kohärenter Lichtquellen belichtet werden können. Dabei bildet sich eine dreidimensionale Struktur in den Photopolymeren aus, die sich im Allgemeinen durch eine regionale Änderung des Brechungsindexes in dem Material beschreiben lässt. Derartige Strukturen werden Hologramme genannt, die auch als diffraktive optische Elemente beschrieben werden können. Dabei hängt es von der speziellen Belictung ab, welche optischen Funktionen ein solches Hologramm ausbildet.

Für die Anwendung von Photopolymeren als Träger von Hologrammen für optische Anwendungen im sichtbaren ($\lambda$ = 400 - 800 nm) sowie im nahen UV Bereich ($\lambda$ = 300 - 400 nm) werden in der Regel nach der Belichtung farblose Materialien mit einer hohen Beugungswirkung gefordert. Seit den Anfängen der Holographie werden Silberhalogenidfilme insbesondere diejenigen mit hohem Auflösungsvermögen hierfür verwendet. Auch Dichromatgelatine (DCG), Dichromatsalz haltige Gelatinefilme, oder auch Mischformen aus Silberhalogenid und DCG werden verwendet. Beide Materialien benötigen eine chemische Nachbehandlung zur Bildung eines Hologramms, was für industrielle Prozesse zusätzliche Kosten erzeugt und den Umgang mit chemischen Entwicklerlösungen erforderlich macht. Zudem haben nasschemische Verfahren ein Quellen und später ein Schrumpfen des Filmes zufolge, was zu Farbverschiebungen in den Hologrammen führen kann, was unerwünscht ist.

US 4959284 (Dupont) beschreibt Photopolymere, die u.a. aus einem in organischen Lösungsmitteln löslichen Thermoplasten wie Polyvinylacetat, Celluloseacetobutyrat oder Polymethylinethacrylat-Styrol-Copolymere, einem Photoinitiator und mindestens einem Vinylcyclopropan bestehen. In EP352774A1 (Dupont) werden zudem andere Vinylgruppen haltigen Monomere beschrieben wie N-Vinylpyrrolidon, Phenoxyethylacrylat und Acrylester von Triolen wie Trimethylolpropan (TMPTA) und ethoxylierten Trimethylolpropan (TMPEOTA) oder andere Acrylester oder Acrylamide. Es ist in der Industrie bekannt, dass derartige Photopolymere erst nach einer längeren Temperaturbehandlung brauchbare Hologramme zeigen. O'Neill et al. (Applied Optics, Vol. 41, No.5, Seite 845ff., 2002) diskutieren in ihrem Übersichtsartikel neben den schon erwähnten Materialen auch noch Photopolymere, die aus Thermoplasten und Acrylamid erhältlich sind. Neben dem ungünstigen toxikologischen Profil von Acrylamid, zeigen derartige Produkte keine hellen Hologramme.

Auch bekannt sind holographisch aktive Materialien, in denen Farbstoffe eingeblendet werden, die unter Lichteinfluss ihre Photoempfindlichkeit verändern (Luo et al, Optics Express, Vol. 13, No.8, 2005, Seite 3123). Ähnlich beschreibt Bieringer (Springer Series in Optical Sciences (2000), 76, Seite 209-228.) sog. photoadressierbare Polymere, die ebenfalls polymergebundene Farbstoffe, die unter Lichteinfluss isomerisiert werden können, enthalten. In beiden Substanzklassen kann man Hologramme einbelichten und diese Materialien zur holographischen Datenspeicherung verwenden. Allerdings sind diese Produkte naturgemäß stark gefärbt und damit nicht für die oben beschriebenen Anwendungen geeignet.

In jüngerer Zeit wurden zudem Photopolymere beschrieben, die nicht aus Thermoplasten, sondern aus vernetzten Polymeren erhalten werden: So ist in US 020070077498 (Fuji) 2,4,6-tribromophenylacrylat beschrieben, das in einer Polyurethanmatrix gelöst ist. US 6103454 (InPhase) beschreibt ebenfalls eine Polyurethanmatrix mit polymerisierbaren Komponenten wie 4-Chlorphenylacrylat, 4-Bromstryrol und Vinylnapthalen. Auch diese Formulierungen wurden für die holographische Datenspeicherung entwickelt, einer holographischen Anwendung, in der man viele, aber auch sehr schwache, nur mit elektronischen Detektoren lesbare Hologramme einschreibt und ausliest. Ihnen gemeinsam ist die Tatsache das hochbrechende photopolymerisierbare Monomere in einer niedrigbrechenderen Matrix gelöst vorliegen. Für optische Anwendungen im gesamten sichtbaren ($\lambda$ = 400 - 800 nm) sowie im nahen UV Bereich ($\lambda$ = 300 - 400 nm) Bereich sind derartige Formulierungen ebenfalls nicht geeignet.

Aufgabe der vorliegenden Erfindung war es, Photopolymere für die Anwendungen als holographische Medien zu entwickeln, die ohne thermische oder nasschemische Nachbehandlung prozessiert werden können und mit denen sich nach der Belichtung farblose Hologramme mit hoher Beugungseffizienz und hoher Helligkeit herstellen lassen.

Neben den physikalischen Eigenschaften sind jedoch auch die Verarbeitbarkeit und Kompatibilität mit anderen Bauteilen wichtig. So spielen organische Materialien, die durch Photopolymerisation, meist als Homo- oder Copolymer hochbrechender Monomere erhalten werden, eine wichtige Rolle, beispielsweise für die Herstellung von optischen Bauteilen wie Linsen, Prismen und optischen Beschichtungen (US 5.916.987) oder für die Erzeugung eines Kontrastes in holographischen Materialien (US 6.780.546). Es besteht für solche und ähnliche Anwendungen Bedarf, den Brechungsindex z.B. durch Zumischen von Komponenten mit hohem oder niedrigem Brechungsindex gezielt einstellen und über Bereiche variieren zu können. Dies kann zu Photopolymeren führen, in denen hochbrechende photopolymerisierbare Monomere in niedrigbrechenden Matrizes gelöst werden, oder umgekehrt niedrigbrechende photopolymerisierbare Monomere in hochbrechenden Matrizes gelöst vorliegen.

Für die vorgenannten Anwendungsgebiete kommen typischerweise Polymerisate olefinisch ungesättigter Verbindungen wie vorzugsweise (Meth)acrylate zum Einsatz. Um einen Brechungsindex von 1,5 und höher zu erreichen, können halogensubstituierte aromatische (Meth)acrylate oder spezielle in US 6.794.471 beschriebene Alkylmethacrylate verwendet werden. Insbesondere letztere sind aufgrund ihrer aufwendigen Herstellung unvorteilhaft.

Die Eignung von substituierten Phenylisocyanat-basierten Urethanacrylaten zur Herstellung entsprechender Polymere wurde von Bowman beschrieben (Polymer 2005, 46, 4735-4742).

Aus der nicht vorveröffentlichten WO-Anmeldung PCT/EP2008/002464 sind (Meth)acrylate mit einem Brechungsindex bei $\lambda$ = 532 nm von mindestens 1.5 bekannt, die sich zur Herstellung von optischen Datenträgern, insbesondere solchen für holographische Speicherverfahren eignen und auf technisch verfügbaren Rohstoffen basieren. In diesem Zusammenhang sind auch Phenylisocyanatbasierte Verbindungen bekannt, wobei diese stets auf unsubstituierten Phenylringen auf der Isocyanatseite basieren.

In Photopolymer-Formulierungen spielen hochbrechende Acrylate als kontrastgebende Komponente (US 6.780.546) eine entscheidende Rolle. Das Interferenzfeld aus Signal- und Referenzlichtstrahl (im einfachsten Fall zwei ebene Wellen) wird durch die lokale Photopolymerisation an Orten hoher Intensität im Interferenzfeld durch die hochbrechenden Acrylate in ein Brechungsindexgitter abgebildet, das alle Information des Signals enthält (das Hologramm). Durch Beleuchtung des Hologramms nur mit dem Referenzlichtstrahl kann dann das Signal wieder rekonstruiert werden. Die maximale Stärke des so rekonstruierten Signals im Verhältnis zur Stärke des eingestrahlten Referenzlichts wird Beugungseffizienz genannt, im folgenden DE wie Diffraction Efficiency. Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die notwendige Lichtmenge des Referenzlichtes die notwendig ist, um das Signal mit einer festen Helligkeit sichtbar zu machen. Hochbrechende Acrylate sind in der Lage, Brechungsindexgitter mit hoher Amplitude $\Delta n$ zwischen Bereichen mit niedrigstem Brechungsindex und Bereichen mit höchstem Brechungsindex zu erzeugen und damit in Photopolymer-Formulierungen Hologramme mit hohem DE zu ermöglichen. (Der Brechungsindexkontrast $\Delta n$ der beim Schreiben eines Volumenhologramms mittels der Überlagerung zweier ebener Wellen resultiert ergibt sich aus folgender Brechungsindexvariation $n(x) = n_0+\Delta n\cdot\cos(K\cdot x)$, wobei K den Betrag des Gittervektor darstellt, der in Richtung der x-Achse zeigt und $n_0$ den mittleren Brechungsindex. Siehe z.B. Hariharan Optical Holography, Principles, Techniques and Applications, Cambridge University Press, 1991 Seite 44).

US 6939648B beschreibt optische Artikel aus Photopolymerformulierungen, die basierend auf einer vernetzten Polyuretharimatrix einen Elastizitätsmodul E von wenigstens 0.1 MPa aufweisen, wobei die Dicke der Photopolymerschicht größer 200 $\mu$m ist. Es wird offenbart, dass je größer der Elastizitätsmodul ist umso bevorzugter die Photopolymerformulierung sein soll. Es wird nicht näher spezifiziert wie der Elastizitätsmodul gemessen wird und wie er in Bezug auf die Topologie und Dynamik der Matrixpolymerstränge zu verstehen ist, d.h. ob er einen gummiartig vernetzen Zustand oder einen glasartig erstarrten Zustand des Photopolymeren charakterisiert. Der Zusammenhang zwischen Vernetzungsdichte, Schreibmonomermolekulargewicht mit der holographischen Performance bei einzelnen starken Hologrammen wird nicht offenbart, insbesondere nicht für Reflexionshologramme. Im Gegenteil führt die in der oben genannten Anmeldung beschriebene Vorzugsrichtung zu höherem Elastizitätsmodul, beim Schreiben einzelner starker Hologramme zu einer Verschlechterung der holographischen Performance, wie an den hier offenbarten Beispielen zu sehen ist.

Eine bekannte Vorgehensweise zur Optimierung der Performance von Photopolymeren in holographischen Anwendungen ist daher die Erhöhung des Unterschiedes zwischen den Brechungsindizes des Matrixpolymers und des darin gelösten Schreibmonomers, z.B. in dem man hochbrechende Schreibmonomere in niedrigbrechenden Matrizes löst oder niedrigbrechende Schreibmonomer in hochbrechenden Matrizes einsetzt.

Bildet man die Matrix als polymeres Netzwerk aus, können die mechanischen, optischen, thermischen und thermodynamischen Eigenschaften des Photopolymeren durch Wahl der das Netzwerk aufbauenden Wiederholungseinheiten und deren Funktionalitäten in weiten Grenzen gezielt eingestellt werden. Im oben beschriebenen Stand der Technik wird nicht offenbart, ob und inwieweit die Vernetzungsdichte solcher Photopolymere die Performance in holographischen Medien entscheidend beeinflussen kann.

Es wurde nun überraschenderweise gefunden, das Photopolymerformulierungen basierend auf einer Matrix, die ein polymeres Netzwerk darstellt und mindestens einem darin gelösten photopolymerisierbaren Monomer besonders dann in holographischen Medien Brechungsindexgitter mit hoher Amplitude ($\Delta n$) zwischen Bereichen mit niedrigstem Brechungsindex und Bereichen mit höchstem Brechungsindex erzeugen, wenn eine niedrige Vernetzungsdichte der Photopolymerformulierung vor der Belichtung vorliegt. Daher sind solche Photopolymerformulierungen besonders geeignet, um in holographischen Medien, wie oben beschrieben helle, visuelle Hologramme mit hoher Beugungseffizienz zu erzeugen. Visuelle Hologramme umfassen alle Hologramme, die nach dem Fachmann bekannten Verfahren aufgezeichnet werden können, darunter fallen unter anderem In-Line (Gabor) Hologramme, Off-Axis Hologramme, Full-Aperture Transfer Hologramme, Weißlicht-Transmissionshologramme ("Regenbogenhologramm"), Denisyukhologramme, Off-Axis Reflektionshologramme, Edge-Lit Hologramme sowie Holographische Stereogramme, bevorzugt sind Reflexionshologramme, Denisyukhologramme, Transmissionshologramme.

Gegenstand der vorliegenden Erfindung sind daher Photopolymerformulierungen umfassend dreidimensional vernetzte organische Polymere A) als Matrix sowie Verbindungen B), die unter Einwirkung aktinischer Strahlung mit ethylenisch ungesättigten Verbindungen unter Polymerisation reagierende Gruppen (strahlenhärtende Gruppen) aufweisen und in dieser Matrix gelöst oder verteilt vorliegen sowie C) wenigstens einen Photoinitiator, gekennzeichnet durch eine Netzwerkdichte des organischen Polymers ausgedrückt durch das mittlere Molekulargewicht $M_C$ der zwei Polymerstränge verbrückenden Segmente von 2685 g/mol bis 55000 g/mol.

Ein weiterer Gegenstand der vorliegenden Erfindung sind holographische Medien, welche aus den erfindungsgemäßen Photopolymerformulierungen erhältlich sind.

Strahlenhärtbare Gruppen im Sinne der vorliegenden Erfindung sind sämtliche funktionelle Gruppen, die unter Einwirkung aktinischer Strahlung mit olefinisch ungesättigten Verbindungen unter Polymerisation reagieren. Dies sind beispielsweise Vinylether- ($CH_2$=CH-O-), Maleinyl- (cis-HOOC-C=C-CO-O-), Fumaryl- (trans-HOOC-C=C-CO-O-), Maleinimid-, Dicyclopentadienyl-, Acrylamid- ($CH_2$=CH-(CO)-NH-), Methacrylamid- ($CH_2=CCH_3$-(CO)-NH-), Acrylat- ($CH_2$=CH-(CO)-O-) und Methacrylat-Gruppen ($CH_2=CCH_3$-(CO)-O-).

Unter aktinischer Strahlung wird elektromagnetische, ionisierende Strahlung verstanden, insbesondere Elektronenstrahlen, UV-Strahlen sowie sichtbares Licht (Roche Lexikon Medizin, 4.Auflage; Urban & Fischer Verlag, München 1999).

Die Bestimmung des mittleren Molekulargewichts $M_c$ erfolgt über die Bestimmung des Plateaumoduls $G_0$ in einem Oszillationsrheometer wobei folgender bekannter Zusammenhang (M. Doi, S.F. Edwards, The Theory of Polymer Dynamics, Oxford Science Publications, 1986) genutzt wird:

$$G_0 = \frac{\rho \cdot R \cdot T}{M_C}$$

R ist die Avogadro Konstante, T die absolute Temperatur in Kelvin und ρ die Massendichte.

Bevorzugt haben die zwei Polymerstränge verbrückenden Segmente mittlere Molekulargewichte $M_c$ von 2685 g/mol bis 55000 g/mol, besonders bevorzugt von 3400 g/mol bis 55000 g/mol, ganz besonders bevorzugt von 7500 g/mol bis 55000 g/mol.

Bevorzugt ist, wenn $M_c$ den vorgenannten Werten entspricht und darüber hinaus das Verhältnis Q von $M_c$ zum zahlenmittleren Molekulargewicht $M_{Mo}$ aller in B) eingesetzten strahlenhärtbaren Verbindungen größer als 3.30, besonders bevorzugt größer als 4.13, ganz besonders bevorzugt größer als 10.00 beträgt.

Neben den Komponenten A) und B) können die erfindungsgemäßen Photopolymerformulierungen Photoinitiatorsysteme C) enthalten, die aus mindestens einer lichtabsorbierenden Komponente und gegebenenfalls mindestens einer weiteren Komponente bestehen, die gegebenenfalls die Energie des angeregten Zustands der lichtabsorbierenden Verbindung aufnimmt und damit den Start der Photopolymerisation auslöst. In geeigneten Systemen kann der Start der Photopolymerisation auch durch die lichtabsorbierende Komponente selbst ausgelöst werden.

Weitere Komponenten können Stabilisatoren sein, die z.B. die Lagerstabilität der Komponenten der Photopolymerformulierung verbessern, oder Stabilisatoren, die z.B. die Stabilität der aus den erfindungsgemäßen Photopolymerformulierungen hergestellten holographischen Medien gegen Umgebungslicht, Temperatur und Feuchte verbessern, oder Additive wie z.B. Lösungsmittel oder wie z.B. Trennmittel, die die Verarbeitung der erfindungsgemäßen Photopolymerformulierungen zu den entsprechenden holographischen Medien erleichtern, bzw. die Nutzbarkeit der holograpischen Medien in der finalen Anwendung verbessern, bzw. erst ermöglichen.

Die Matrix (Komponente A) ist ein festes Polymer mit dreidimensionaler Netzwerkstruktur, das *in situ* aus der Reaktion einer oder mehrerer Vorstufen durch einen "curing step" geformt wird. Dabei wird die Reaktion zur Ausbildung der Matrix durch eine Initiierungsreaktion ausgelöst. Die Vorstufen können aus einer Sorte Monomer, mehreren Monomeren, einer Sorte Oligomeren, mehreren Oligomeren oder einer Mischung von Monomeren und Oligomeren bestehen. Außerdem ist es möglich, dass eine oder mehrere der Vorstufen mehr als eine Sorte im "curing step" reagierende funktionelle Gruppe tragen. Um eine gute Mischbarkeit der Vorstufe(n) mit den anderen Bestandteilen der Formulierung zu gewährleisten, ist diese bevorzugt flüssig in einem gewissen Temperaturbereich zwischen -50°C und 80°C. Besonders bevorzugt lässt sich die Mischung bei Temperaturen zwischen 15°C und 75°C in einer Zeitdauer von kleiner 200 Minuten herstellen. Die Matrix habe eine Glasübergangstemperatur, die klein genug ist, um während des Schreibens des Hologramms chemische Reaktionen sowie eine genügende Diffusion der Komponente B) zuzulassen. Bevorzugt ist ein Temperaturbereich zwischen -130°C und 80°C. Beispiele chemischer Reaktionen zum Aufbau einer solchen Matrix sind die kationische Epoxidpolymerisation, die kationische Polymerisation von Vinylethern, die kationische Polymerisation

von Alkenylethern, die kationische Allenpolymerisation, die kationische Keten-Acetal-Polymerisation, die Additionspolymerisation von Epoxiden und Aminen bzw. Epoxiden und Thiolen, die Poly-Michaeladdition (Additionspolymerisation ungesättigter Ester mit Aminen oder Thiolen), die Additionspolymerisation von Siliconhydriden mit Vinylverbindungen über Hydrosilylierung sowie die Polyaddition von Isocyanaten an OH- oder NH-funktionelle Verbindungen (durch Polyurethan- oder Polyharnstoffbildung). Verschiedene der beschriebenen Reaktionen lassen sich durch die Gegenwart geeigneter Katalysatoren beschleunigen.

Bevorzugt sind solche dreidimensional vernetzten organischen Polymere, die Urethangruppen aufweisen.

Besonders bevorzugt sind solche dreidimensional vernetzte organische Polymere, welche als Vorstufen aus einer Isocyanatkomponente a) und einer Isocyanat-reaktiven Komponente b) aufgebaut sind.

Als Verbindungen der Polyisocyanatkomponente a) eignen sich alle dem Fachmann an sich bekannten aliphatischen, cycloaliphatischen, aromatischen oder araliphatischen Di- und Triisocyanate, wobei es unerheblich ist, ob diese mittels Phosgenierung oder nach phosgenfreien Verfahren erhalten wurden. Daneben können auch die dem Fachmann an sich gut bekannten höhermolekularen Folgeprodukte (Oligo- und Polyisocyanate) monomerer Di- und/oder Triisocyanate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Beispielsweise geeignete monomere Di- oder Triisocyanate sind Butylendiisocyanat, Hexamethylendiisocyanat (HDI), Isophorondiisocyanat (IPDI), Trimethyl-hexamethylen-diisocyanat (TMDI), 1,8-Diisocyanato-4-(isocyanatomethyl)oktan, Isocyanatomethyl-1,8-octandiisocyanat (TIN), 2,4-und/oder 2,6-Toluen-diisocyanat.

Ebenfalls möglich ist der Einsatz von isocyanatfunktionellen Prepolymeren mit Urethan-, Allophanat- oder Biuretstrukturen als Verbindungen der Komponente a), wie sie in an sich gut bekannter Art und Weise durch Umsetzung der vorgenannten Di-, Tri- oder Polyisocyanate im Überschuss mit hydroxy- oder aminofunktionellen Verbindungen erhalten werden können. Eventuell nicht umgesetztes Ausgangsisocyanat kann anschließend noch entfernt werden, um monomerenarme Produkte zu erhalten. Zur Beschleunigung der Prepolymerbildung kann der Einsatz von dem Fachmann an sich aus der Polyurethanchemie gut bekannten Katalysatoren hilfreich sein.

Als hydroxy- oder aminofunktionellen Verbindungen zum Prepolymeraufbau eignen sich typischerweise niedermolekulare kurzkettige, d.h. 2 bis 20 Kohlenstoffatome enthaltende aliphatische, araliphatische oder cycloaliphatische Diole, Triole und/oder höhere Polyole.

Beispiele für Diole sind Ethylenglykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethylpentandiol, stellungsisomere Diethyloctandiole, 1,3-Butylenglykol, Cyclohexandiol, 1,4-Cyclohexandimethanol, 1,6-Hexandiol, 1,2- und 1,4-Cyclohexandiol, hydriertes Bisphenol A (2,2-Bis(4-hydroxycyclohexyl)propan), 2,2-Dimethyl-3-hydroxypropionsäure(2,2-dimethyl-3-hydroxypropylester).

Beispiele geeigneter Triole sind Trimethylolethan, Trimethylolpropan oder Glycerin. Geeignete höherfunktionelle Alkohole sind Ditrimethylolpropan, Pentaerythrit, Dipentaerythrit oder Sorbit.

Geeignet sind auch höhermolekulare aliphatische und cycloaliphatische Polyole wie Polyesterpolyole, Polyetherpolyole, Polycarbonatpolyole, hydroxyfunktionelle Acrylharze, hydroxyfunktionelle Polyurethane, hydroxyfunktionelle Epoxyharze oder entsprechende Hybride (vgl. Römpp Lexikon Chemie, S.465-466, 10. Aufl. 1998, Georg-Thieme-Verlag, Stuttgart).

Für den Prepolymeraufbau geeignete Polyesterpolyole sind lineare Polyesterdiole, wie sie in bekannter Weise aus aliphatischen, cycloaliphatischen oder aromatischen Di- bzw. Polycarbonsäuren bzw. ihren Anhydriden wie z. B. Bernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren Gemisch mit mehrwertigen Alkoholen wie z. B. Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12 oder deren Gemische gegebenenfalls unter Mitverwendung höherfunktioneller Polyole wie Trimethylolpropan oder Glycerin hergestellt werden können. Als mehrwertige Alkohole zur Herstellung der Polyesterpolyole kommen natürlich auch cycloaliphatische und/oder aromatische Di- und Polyhydroxylverbindungen in Frage. Anstelle der freien Polycarbonsäure können auch die entsprechenden Polycarbonsäureanhydride oder entsprechende Polycarbonsäureester von niedrigen Alkoholen oder deren Gemische zur Herstellung der Polyester verwendet werden.

Ebenfalls zum Prepolymeraufbau geeignete Polyesterpolyole sind Homo- oder Mischpolymerisate von Lactonen, die vorzugsweise durch Anlagerung von Lactonen bzw. Lactongemischen wie Butyrolacton, ε-Caprolacton und/oder Methyl-ε-caprolacton an geeignete di- und /oder höherfunktionelle Startermoleküle, wie z. B. die vorstehend als Aufbaukomponenten für Polyesterpolyole genannten niedermolekularen, mehrwertigen Alkohole, erhalten werden.

Auch Hydroxylgruppen aufweisenden Polycarbonate kommen als Polyhydroxylkomponente für den Prepolymeraufbau in Betracht, z. B. solche, die durch Umsetzung von Diolen wie 1,4-Butandiol und/oder 1,6-Hexandiol und/oder 3-Methylpentandiol mit Diarylcarbonaten, z. B. Diphenylcarbonat, Dimethylcarbonat oder Phosgen hergestellt werden können.

Zum Prepolymeraufbau geeignete Polyetherpolyole sind z. B. die Polyadditionsprodukte der Styroloxide, des Ethylenoxid, Propylenoxid, Tetrahydrofuran, Butylenoxid, Epichlorhydrins, sowie ihre Mischadditions- und Pfropfprodukte, sowie die durch Kondensation von mehrwertigen Alkoholen oder Mischungen derselben und die durch Alkoxylierung von mehrwertigen Alkoholen, Aminen und Aminoalkoholen gewonnenen Polyetherpolyole. Bevorzugte Polyetherpolyole sind Poly(propylenoxid)e, Poly(ethylenoxide) und deren Kombinationen in Form von statistischen oder Blockcopolymeren oder Poly(tetrahydrofuran)e sowie Mischungen derselben mit einer OH-Funktionalität von 1,5 bis 6 und einem zahlenmittleren Molekulargewicht von zwischen 200 bis 18000 g/mol, bevorzugt mit einer OH-Funktionalität von 1,8 bis 4,0 und einem zahlenmittleren Molekulargewicht von 600 bis 8000 g/mol und besonders bevorzugt mit einer OH-Funktionalität von 1,9 bis 3,1 und einemr zahlenmittleren Molekulargewicht von 650 bis 4500 g/mol.

Als Amine eignen sich für den Prepolymeraufbau alle oligomeren oder polymeren, primären oder sekundären, di-, tri- oder polyfunktionellen Amine. Dies können beispielsweise sein: Ethylendiamin, Diethylentriamin, Triethylentetramin, Propylendiamin, Diamino-cyclohexan, Diaminobenzol, Diaminobisphenyl, Triaminobenzol, di-, tri- und höherfunktionelle Polyamine wie z.B. die Jeff-amine®, aminterminierte Polymere mit zahlenmittleren Molmassen bis 10000 g/mol oder deren beliebige Gemische untereinander.

Bevorzugte Prepolymere sind solche basierend auf den vorgenannten Aufbaukomponenten mit Urethan- und/oder Allophanatgruppen mit zahlenmittleren Molekulargewichten von 200 bis 10000 g/mo, bevorzugt mit zahlenmittleren Molekulargewichten von 500 bis 8000 g/mol. Besonders bevorzugte Prepolymere sind Allophanate basierend auf HDI oder TMDI und di- oder trifunktionellen Polyetherpolyolen mit zahlenmittleren Molmassen von 1000 bis 8000 g/mol.

Es ist gegebenenfalls auch möglich, dass die Isocyanatkomponente a) anteilsmäßig Isocyanate enthält, die teilweise mit isocyanat-reaktiven ethylenisch ungesättigten Verbindungen umgesetzt sind. Bevorzugt werden hierbei als isocyanat-reaktive ethylenisch ungesättigte Verbindungen $\alpha,\beta$-ungesättigte Carbonsäurederivate wie Acrylate, Methacrylate, Maleinate, Fumarate, Maleimide, Acrylamide, sowie Vinylether, Propenylether, Allylether und Dicyclopentadienyl-Einheiten enthaltende Verbindungen, die mindestens eine gegenüber Isocyanaten reaktive Gruppe aufweisen, eingesetzt. Besonders bevorzugt sind Acrylate und Methacrylate mit mindestens einer isocyanatreaktiven Gruppe. Als hydroxyfunktionelle Acrylate oder Methacrylate kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)acrylate, Poly ("epsilon"-caprolacton)-mono(meth)acrylate, wie z.B. Tone® M100 (Dow, USA), 2-Hydroxypropyl(meth)acrylat, 4-Hydroxybutyl(meth)acrylat, 3-Hydroxy-2,2-dimethylpropyl(meth)acrylat, die hydroxyfunktionellen Mono-, Di- oder Tetra (meth)acrylate mehrwertiger Alkohole wie Trimethylolpropan, Glycerin, Pentaerythrit, Dipentaerythrit, ethoxiliertes, propoxiliertes oder alkoxyliertes Trimethylolpropan, Glycerin, Pentaerythrit, Dipentaerythrit oder deren technische Gemische in Betracht. Darüberhinaus sind isocyanat-reaktive oligomere oder polymere ungesättigte Acrylat- und/oder Methacrylatgruppen enthaltende Verbindungen alleine oder in Kombination mit den vorgenannten monomeren Verbindungen geeignet. Der Anteil an Isocyanaten, die teilweise mit isocyanat-reaktiven ethylenisch ungesättigten Verbindungen umgesetzt sind, an der Isocyanatkomponente a) beträgt 0 bis 99 %, bevorzugt 0 bis 50 %, besonders bevorzugt 0 bis 25 % und ganz besonders bevorzugt 0 bis 15%.

Die NCO-Gruppen der Polyisocyanate der Komponente a) können auch ganz oder teilweise mit den an sich in der Technik üblichen Blockierungsmitteln blockiert sein. Dies sind beispielsweise Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, epsilon-Caprolactam, N-tert.-Butyl-benzylamin, Cyclopentanoncarboxyethylester oder beliebige Gemische dieser Blockierungsmittel.

Bevorzugt werden in Komponente a) Polyisocyanate und/oder Präpolymere der vorstehend genannten Art basierend auf HDI, TMDI und/oder TIN eingesetzt.

Besonders bevorzugt werden Polyisocyanate basierend auf HDI mit Isocyanurat- und/oder Iminooxadiazindionstrukturen eingesetzt.

Ebenfalls besonders bevorzugt ist der Einsatz von Präpolymeren bevorzugt mit NCO-Funktionalitäten von 2 bis 5, besonders bevorzugt solchen mit primären NCO-Gruppen. Beispiele solcher Präpolymere sind Allophanate oder Urethane oder deren Mischungen bevorzugt auf Basis von HDI und/oder TMDI und Polyether- und/oder Polyester- bzw. Polycarbonatpolyolen. Bevorzugt weisen die vorstehend genannten Polyisocyanate bzw. Präpolymere Restgehalte an freiem monomeren Isocyanat von weniger als 1 Gew.-%, besonders bevorzugt weniger als 0,5 Gew.-%, ganz besonders bevorzugt weniger als 0,2 Gew.-% auf.

Als Komponente b) können an sich alle polyfunktionellen, isocyanatreaktiven Verbindungen eingesetzt werden, die im Mittel wenigstens 1.5 isocyanatreaktive-Gruppen pro Molekül aufweisen.

Isocyanatreaktive Gruppen im Rahmen der vorliegenden Erfindung sind bevorzugt Hydroxy-, A-mino- oder Thiogruppen, besonders bevorzugt sind Hydroxyverbindungen.

Geeignete polyfunktionelle, isocyanatreaktive Verbindungen sind beispielsweise Polyester-, Polyether-, Polycarbonat-, Poly(meth)acrylat- und/oder Polyurethanpolyole.

Als Polyesterpolyole sind beispielsweise lineare Polyesterdiole oder verzweigte Polyesterpolyole geeignet, wie sie in bekannter Weise aus aliphatischen, cycloaliphatischen oder aromatischen Di- bzw. Polycarbonsäuren bzw. ihren Anhydriden mit mehrwertigen Alkoholen einer OH-Funktionalität ≥ 2 erhalten werden.

Beispiele für solche Di- bzw. Polycarbonsäuren bzw. Anhydride sind Bernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren beliebige Gemische untereinander.

Beispiele für solche geeigneten Alkohole sind Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12, Trimethylolpropan, Glycerin oder deren beliebige Gemische untereinander.

Die Polyesterpolyole können auch auf natürlichen Rohstoffen wie Rizinusöl basieren. Ebenfalls möglich ist, dass die Polyesterpolyole auf Homo- oder Mischpolymerisaten von Lactonen basieren, wie sie bevorzugt durch Anlagerung von Lactonen bzw. Lactongemischen wie Butyrolacton, ε-Caprolacton und/oder Methyl-ε-caprolacton an hydroxyfunktionelle Verbindungen wie mehrwertige Alkohole einer OH-Funktionalität ≥ 2 beispielsweise der vorstehend genannten Art erhalten werden können.

Solche Polyesterpolyole haben bevorzugt zahlenmittlere Molmassen von 400 bis 8000 g/Mol, besonders bevorzugt von 500 bis 4000 g/Mol. Ihre OH-Funktionalität beträgt bevorzugt 1.5 bis 3.5, besonders bevorzugt 1.8 bis 3.0.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkoholen einer OH-Funktionalität ≥ 2, bevorzugt 1,4-Butandiol, 1,6-Hexandiol und/oder 3-Methylpentandiol, oder auch Polyesterpolyole können zu Polycarbonatpolyolen umgearbeitet werden.

Solche Polycarbonatpolyole haben bevorzugt zahlenmittlere Molmassen von 400 bis 4000 g/Mol, besonders bevorzugt von 500 bis 2400 g/Mol. Die OH-Funktionalität dieser Polyole beträgt bevorzugt 1.8 bis 3.2, besonders bevorzugt 1.9 bis 3.0.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin, sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Aminoalkohole verwendet werden.

Solche Polyetherpolyole haben bevorzugt zahlenmittlere Molmassen von 250 bis 10000 g/Mol, besonders bevorzugt von 500 bis 8500 g/Mol und ganz besonders bevorzugt von 600 bis 4500 g/Mol. Die OH-Funktionalität beträgt bevorzugt 1.5 bis 4.0, besonders bevorzugt 1.8 bis 3.0.

Daneben sind als Bestandteile der Komponente b) als polyfunktionelle, isocyanatreaktive Verbindungen auch niedermolekulare, d.h. mit Molekulargewichten kleiner 500 g/mol, kurzkettige, d.h. 2 bis 20 Kohlenstoffatome enthaltende aliphatische, araliphatische oder cycloaliphatische di, tri oder polyfunktionelle Alkohole geeignet.

Dies können beispielsweise sein Ethylenglykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethylpentandiol, stellungsisomere Diethyloctandiole, 1,3-Butylenglykol, Cyclohexandiol, 1,4-Cyclohexandimethanol, 1,6-Hexandiol, 1,2- und 1,4-Cyclohexandiol, hydriertes Bisphenol A (2,2-Bis(4-hydroxycyclohexyl)propan), 2,2-Dimethyl-3-hydroxypropionsäure (2,2-dimethyl-3-hydroxypropylester). Beispiele geeigneter Triole sind Trimethylolethan, Trimethylolpropan oder Glycerin. Geeignete höherfunktionelle Alkohole sind Ditrimethylolpropan, Pentaerythrit, Dipentaerythrit oder Sorbit.

Bevorzugte Komponenten b) sind Polyetherpolyole, Poly(propylenoxid)e, Poly(ethylenoxide) und deren Kombinationen in Form von statistischen oder Blockcopolymeren sowie Blockcopolymere aus Propylenoxid und/oder Ethylenoxid, die zusätzlich Tetrahydrofuran, Butylenoxid oder ε-Caprolacton als Monomereinheiten enthalten sowie Mischungen derselben mit einer OH-Funktionalität von 1,5 bis 6 und einer zahlenmittleren Molmasse zwischen 200 und 18000 g/Mol, besonders bevorzugt mit einer OH-Funktionalität von 1,8 bis 4,0 und einer zahlenmittleren Molmasse zwischen 600 und 8000 g/Mol und ganz besonders bevorzugt mit einer OH-Funktionalität von 1,9 bis 3,1 und einer zahlenmittleren Molmasse zwischen 650 und 4500 g/Mol.

Weiterhin bevorzugt zur Herstellung der Matrix A) werden Kombinationen aus oben benannten Isocyanatkomponenten a) und isocyanatreaktive Komponenten b), die möglichste hohes Molekulargewicht zwischen den entsprechenden funktionellen Gruppen besitzen und/oder möglichst geringe Funktionalitäten aufweisen, wobei die Funktionalitäten aber groß genug sein müssen, um ein dreidimensionales Netzwerk aufbauen zu können.

Ferner sind auch solche Kombinationen bevorzugt bei denen die funktionellen Gruppen der isocyanatreaktiven

Komponenten b) im molaren Überschuss zu der funktionellen Gruppen der Isocyanatkomponenten a) vorliegen.

Bevorzugt werden in Komponente B) Verbindungen mit Vinylether-, Acrylat- oder Methacrylat-Gruppe, besonders bevorzugt Acrylat- und/oder Methacrylatgruppen eingesetzt.

Bevorzugt werden in B) Verbindungen der vorstehend genannten Art mit einem Brechungsindex $n_D^{20}$ von größer 1.54, bevorzugt von größer 1.55 und besonders bevorzugt von größer 1.58 eingesetzt.

Bevorzugt werden in B) Verbindungen der vorstehenden genannten Art mit Molekulargewichten kleiner als 1500 g/mol, bevorzugt kleiner als 1000 g/mol.

In Komponente B) können Verbindungen wie α,β-ungesättigte Carbonsäurederivate wie Acrylate, Methacrylate, Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol, Olefinine, wie z.B. 1-Octen und/oder 1-Decen, Vinylestern, (Meth)acrylnitril, (Meth)acrylamid, Methacrylsäure, Acrylsäure eingesetzt werden. Bevorzugt sind Acrylate und Methacrylate.

Als Acrylate bzw. Methacrylate werden allgemein Ester der Acrylsäure bzw. Methacrylsäure bezeichnet. Beispiele verwendbarer Acrylate und Methacrylate sind Methylacrylat, Methylmethacrylat, Ethylacrylat, Ethylmethacrylat, Ethoxyethylacrylat, Ethoxyethylmethacrylat, n-Butylacrylat, n-Butylmethacrylat, tert.-Butylacrylat, tert.-Butylmethacrylat, Hexylacrylat, Hexylmethacrylat, 2-Ethylhexylacrylat, 2-Ethylhexylmethacrylat, Butoxyethylacrylat, Butoxyethylmethacrylat, Laurylacrylat, Laurylmethacrylat, Isobornylacrylat, Isobornylmethacrylat, Phenylacrylat, Phenylmethacrylat, p-Chlorphenylacrylat, p-Chlorphenylmethacrylat, p-Bromphenylacrylat, p-Bromphenylmethacrylat, 2,4,6-Trichlorphenylacrylat, 2,4,6-Trichlorphenylmethacrylat, 2,4,6-Tribromphenylacrylat, 2,4,6-Tribromphenylmethacrylat, Pentachlorphenylacrylat, Pentachlorphenylmethacrylat, Pentabromphenylacrylat, Pentabromphenylmethacrylat, Pentabrombenzylacrylat, Pentabrombenzylmethacrylat, Phenoxyethylacrylat, Phenoxyethylmethacrylat, Phenoxyethoxyethylacrylat, Phenoxyethoxyethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Propan-2,2-diylbis-[(2,6-dibrom-4,1-phenylen)oxy(2-{[3,3,3-tris(4-chlorphenyl)-propanoyl]-oxy}propan-3,1-diyl)oxy-ethan-2,1-diyl]-diacrylat, Bisphenol A Diacrylat, Bisphenol A Dimethacrylat, Tetrabromobisphenol A Diacrylat, Tetrabromobisphenol A Dimethacrylat sowie deren ethoxylierte Analogverbindungen, N-Carbazolylacrylate um nur eine Auswahl verwendbarer Acrylate und Methacrylate zu nennen.

Selbstverständlich können auch Urethanacrylate als Komponente B) verwendet werden. Unter Urethanacrylaten versteht man Verbindungen mit mindestens einer Acrylsäureestergruppe die zusätzlich über mindestens eine Urethanbindung verfügen. Es ist bekannt, dass solche Verbindungen durch Umsetzung eines Hydroxy-funktionellen Acrylsäureesters mit einer Isocyanatfunktionellen Verbindung erhalten werden können.

Beispiele hierfür verwendbarer Isocyanate sind aromatische, araliphatische, aliphatische und cycloaliphatische Di-, Tri- oder Polyisocyanate. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisocyanat (HDI), Isophorondiisocyanat (IPDI), 1,8-Diisocyanato-4-(isocyanatomethyl)oktan, 2,2,4- und/oder 2,4,4-Trimethylhexamethylendiisocyanat, die isomeren Bis(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, 1,5-Naphthylendiisocyanat, Triphenylmethan-4,4',4''-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische oder araliphatische Di-, Tri- oder Polyisocyanate.

Als hydroxyfunktionelle Acrylate oder Methacrylate für die Herstellung von Urethanacrylaten kommen beispielsweise in Betracht Verbindungen wie 2-Hydroxyethyl(meth)acrylat, Polyethylenoxid-mono(meth)acrylate, Polypropylenoxidmono(meth)acrylate, Polyalkylenoxidmono(meth)-acrylate, Poly(ε-caprolacton)mono(meth)acrylate, wie z.B. Tone® M100 (Dow, Schwalbach, DE), 2-Hydroxypropyl(meth)acrylat, 4-Hydroxybutyl(meth)acrylat, 3-Hydroxy-2,2-dimethyl-propyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxiliertes, propoxiliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylate. Darüberhinaus sind als isocyanat-reaktive oligomere oder polymere ungesättigte Acrylat- und/oder Methacrylatgruppen enthaltende Verbindungen alleine oder in Kombination mit den vorgenannten monomeren Verbindungen geeignet. Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth) acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten. Solche Verbindungen werden ebenfalls in P. K. T. Oldring (Ed.), Chemistry & Technology of UV & EB Formulations For Coatings, Inks & Paints, Vol. 2, 1991, SITA Technology, London S 37 - 56 beschrieben.

Bevorzugt sind hydroxylgruppenhaltige Epoxyacrylate mit definierter Hydroxyfunktionalität. Hydroxylguppenhaltige Epoxy(meth)acrylate basieren insbesondere auf Umsetzungsprodukten von Acrylsäure und/oder Methacrylsäure mit Epoxiden (Glycidylverbindungen) von monomeren, oligomeren oder polymeren Bisphenol-A, Bisphenol-F, Hexandiol und/oder Butandiol oder deren ethoxilierten und/oder propoxilierten Derivaten. Bevorzugt sind weiterhin Epoxyacrylate mit definierter Funktionalität wie sie aus der bekannten Umsetzung von Acrylsäure und/oder Methacrylsäure und Glycidyl (meth)acrylat erhalten werden können.

In einer besonders bevorzugten Ausführungsform der Erfindung umfasste die Schreibmonomerkomponente B) ein oder mehrere Verbindungen der Formeln (I) bis (III):

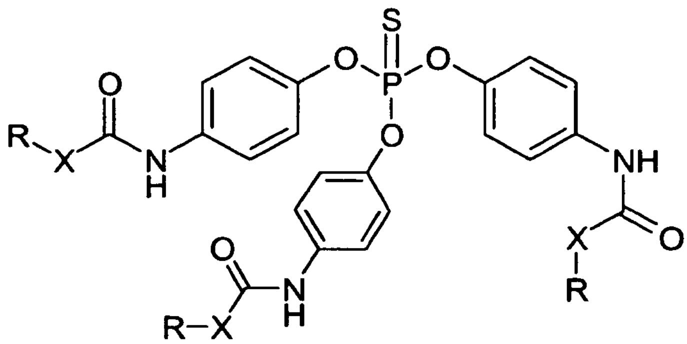

Formel (I)

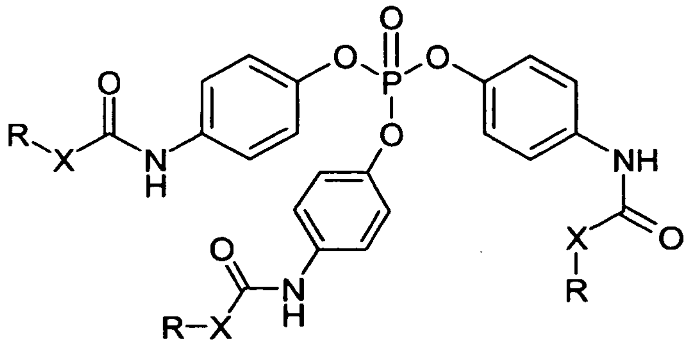

Formel (II)

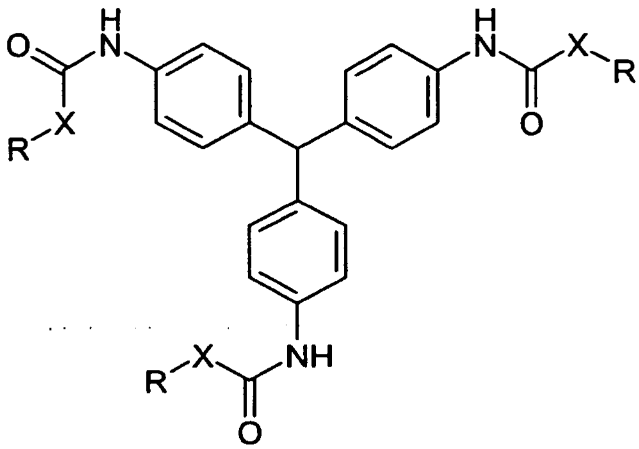

Formel (III)

wobei

R unabhängig voneinander jeweils eine strahlenhärtbare Gruppe und

X unabhängig voneinander jeweils eine Einfachbindung zwischen R und C=O oder ein linea- rer, verzweigter oder cyclischer gegebenenfalls heteroatomhaltiger und/oder gegebenen- falls durch funktionelle Gruppen substituierter Kohlenwassertoffrest ist.

Bevorzugt ist R eine Vinylether-, Acrylat- oder Methacrylat-Gruppe, besonders bevorzugt eine Acrylatgruppe.
Grundsätzlich können einzelne oder mehrere der kohlenstoffgebundenen Wasserstoffatome der Gruppe R auch durch $C_1$- bis $C_5$-Alkylgruppen ersetzt sein, was allerdings nicht bevorzugt ist.
Bevorzugt weist die Gruppe X 2 bis 40 Kohlenstoffatome sowie ein oder mehrere in Form von Etherbrücken vorliegende Sauerstoffatome auf. X kann dabei sowohl linear als auch verzweigt oder cyclisch sowie durch funktionelle Gruppen substituiert sein. Besonders bevorzugt ist die Gruppe X jeweils eine lineare oder verzweigte Oxyalkylen- oder Polyoxyalkylengruppe.
Bevorzugte Polyoxyalkylengruppen weisen bis zu 10, bevorzugt bis zu 8 Wiederholungseinheiten der jeweiligen Oxyalkylengruppe auf.
Grundsätzlich ist es möglich, dass X gleiche oder verschiedene Oxyalkylengruppen als Wiederholungseinheiten aufweist, wobei eine solche Wiederholungseinheit bevorzugt 2 bis 6, besonders bevorzugt 2 bis 4 Kohlenstoffatome aufweist. Besonders bevorzugte Oxyalkyleneinheiten sind Oxyethylen sowie jeweils die isomeren Oxypropylene oder Oxybutylene.
Die Wiederholungseinheiten innerhalb der jeweiligen Gruppe X können dabei ganz oder teilweise blockweise oder statistisch verteilt vorliegen.
In einer bevorzugten Ausführungsform der Erfindung ist X unabhängig voneinander jeweils eine Oxyalkyleneinheit ausgewählt aus der Gruppe bestehend aus $-CH_2-CH_2-O-$, $-CH_2-CHCH_3-O-$, $-CHCH_3-CH_2-O-$, $-(CH_2-CH_2-O)_n-$, $-O(CH_2-CHCH_3-O)_n-$ wobei n eine ganze Zahl von 2 bis 7 ist, und $-O-CH_2-CH_2-(O-(CH_2)_5-CO)_m-$, wobei m eine ganze Zahl von 1 bis 5 ist.
Als Komponente C) werden ein oder mehrere Photoinitiatoren eingesetzt. Dies sind üblicherweise durch aktinische Strahlung aktivierbare Initiatoren, die eine Polymerisation der entsprechenden polymerisierbaren Gruppen auslösen. Photoinitiatoren sind an sich bekannte, kommerziell vertriebene Verbindungen, wobei zwischen unimolekularen (Typ I) und bimolekularen (Typ II) Initiatoren unterschieden wird. Desweiteren werden diese Initiatoren je nach chemischer Natur für die radikalische, die anionische (oder), die kationische (oder gemischte) Formen der vorgenannten Polymerisationen eingesetzt.
(Typ I)-Systeme für die radikalische Photopolymerisation sind z.B. aromatische Ketonverbindungen, z.B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacyclophosphinoxid, Phenylglyoxylsäureester, Campherchinon, alpha-Aminoalkylphenon, alpha-,alpha-Dialkoxyacetophenon, 1-[4-(Phenylthio)phenyl]octan-1,2-dion-2-(O-benzoyloxim) und alpha-Hydroxyalkylphenon. Auch die in EP-A 0223587 beschriebenen Photoinitiatorsysteme bestehend aus einer Mischung aus einem Ammoniumarylborat und einem oder mehreren Farbstoffen können als Photoinitiator eingesetzt werden. Als Ammoniumarylborat eignen sich beispielsweise Tetrabutylammonium Triphenylhexylborat, Tetrabutylammonium Tris-(3-fluorphenyl)-hexylborat und Tetrabutylammonium Tris-(3-Chlor-4-methylphenyl)-hexylborat. Als Farbstoffe eignen sich beispielsweise Neu-Methylenblau, Thionin, Basic Yellow, Pinacynol Chlorid, Rhodamin 6G, Gallocyanin, Ethylviolett, Victoria Blue R, Celestine Blue, Chinaldinrot, Kristallviolett, Brilliant Grün, Astrazon Orange G, Darrow Red, Pyronin Y, Basic Red 29, Pyrillium I, Cyanin und Methylenblau, Azur A (Cunningham et al., RadTech'98 North America UV/EB Conference Proceedings, Chicago, Apr. 19-22, 1998).
Die für die anionische Polymerisation verwendeten Photoinitiatoren sind in der Regel (Typ I)-Systeme und leiten sich von Übergangsmetall-Komplexen der ersten Reihe ab. Hier sind ChromSalze, wie z.B. trans-$Cr(NH_3)_2(NCS)_4^-$ (Kutal et al, Macromolecules 1991, 24, 6872) oder Ferrocenyl-Verbindungen (Yamaguchi et al. Macromolecules 2000, 33, 1152) bekannt. Eine weitere Möglichkeit der anionischen Polymerisation besteht in der Verwendung von Farbstoffen, wie Kristallviolett Leukonitril oder Malchit Grün Leukonitril, die durch photolytischen Zerfall Cyanoacrylate polymerisieren können (Neckers et al. Macromolecules 2000, 33, 7761). Allerdings wird dabei das Chromophor in das Polymer eingebaut, so dass die resultierenden Polymere durchgefärbt sind.
Die für die kationische Polymerisation verwendeten Photoinitiatoren bestehen im wesentlichen aus drei Klassen: Aryldiazonium-Salze, Onium-Salze (hier speziell: Iodonium-, Sulfonium- und Selenonium-Salze) sowie Organometall-Verbindungen. Phenyldiazonium-Salze können unter Bestrahlung sowohl in Gegenwart als auch in Abwesenheit eines Wasserstoff-Donors ein Kation erzeugen, das die Polymerisation initiiert. Die Effizienz des Gesamtsystems wird durch die Natur des verwendeten Gegenions zur Diazonium-Verbindung bestimmt. Bevorzugt sind hier die wenig reaktiven aber recht teuren $SbF_6^-$, $AsF_6^-$ oder $PF_6^-$. Für den Einsatz in Beschichtung dünner Filme sind diese Verbindungen i.d.R

wenig geeignet, da durch den nach der Belichtung freigesetzten Stickstoff die Oberflächegüte herabgesetzt wird (pinholes) (Li et al., Polymeric Materials Science and Engineering, 2001, 84, 139). Sehr weit verbreitet und auch in vielerlei Form kommerziell erhältlich sind Onium-Salze, speziell Sulfonium- und Iodonium-Salze. Die Photochemie dieser Verbindungen ist nachhaltig untersucht worden. Die Iodonium-Salze zerfallen nach der Anregung zunächst homolytisch und erzeugen somit ein Radikal und ein Radikalkation, welches sich durch H-Abstraktion stabilisiert, ein Proton freisetzt und dann die kationische Polymerisation startet (Dektar et al. J. Org. Chem. 1990, 55, 639; J. Org. Chem., 1991, 56. 1838). Dieser Mechanismus ermöglicht den Einsatz von Iodonium-Salzen ebenfalls für die radikalische Photopolymerisation. Hierbei kommt erneut der Wahl des Gegenions eine große Bedeutung zu, bevorzugt werden ebenfalls die recht teuren $SbF_6^-$, $AsF_6^-$ oder $PF_6^-$. Ansonsten ist in dieser Strukturklasse die Wahl der Substitution des Aromaten recht frei und im wesentlichen durch die Verfügbarkeit geeigneter Startbausteine für die Synthese bestimmt. Bei den Sulfonium-Salzen handelt es sich um Verbindungen, die nach Norrish(II) zerfallen (Crivello et al., Macromolecules, 2000, 33, 825). Auch bei den Sulfonium-Salzen kommt der Wahl des Gegenions eine kritische Bedeutung zu, die sich im Wesentlichen in der Härtungsgeschwindigkeit der Polymere äußert. Die besten Ergebnisse werden i.d.R. mit $SbF_6^-$ Salzen erzielt. Da die Eigenabsorption von Iodonium- und Sulfonium-Salze bei <300nm liegt, müssen diese Verbindungen für die Photopolymerisation mit nahem UV oder kurzwelligem sichtbarem Licht entsprechend sensibilisiert werden. Dies gelingt durch die Verwendung von höher absorbierenden Aromaten wie z.B. Anthracen und Derivaten (Gu et al., Am. Chem. Soc. Polymer Preprints, 2000, 41 (2), 1266) oder Phenothiazin bzw. dessen Derivaten (Hua et al, Macromolecules 2001, 34, 2488-2494).

Es kann vorteilhaft sein auch Gemische dieser Verbindungen einzusetzen. Je nach zur Härtung verwendeter Strahlungsquelle muss Typ und Konzentration an Photoinitiator in dem Fachmann bekannter Weise angepasst werden. Die vorgenannte Einstellung hinsichtlich der Photopolymerisation ist für einen Fachmann in Form von Routineversuchen innerhalb der unten angegebenen Mengenbereiche der Komponenten sowie der jeweils zur Auswahl stehenden, insbesondere den bevorzugten Aufbaukomponenten, leicht möglich.

Bevorzugte Photoinitiatoren C) sind Mischungen aus Tetrabutylammonium Tetrahexylborat, Tetrabutylammonium Triphenylhexylborat, Tetrabutylammonium Tris-(3-fluorphenyl)-hexylborat und Tetrabutylammonium Tris-(3-Chlor-4-methylphenyl)-hexylborat mit Farbstoffen wie beispielsweise Astrazon Orange G, Methylenblau, Neu Methylenblau, Azur A, Pyrillium I, Safranin O, Cyanin, Gallocyanin, Brilliant Grün, Kristallviolett, Ethylviolett und Thionin.

Ferner können die erfindungsgemäßen Formulierungen neben den Komponenten A) bis C) auch Radikalstabilisatoren, Katalysatoren und weitere Zusatzstoffe mit eingesetzt werden.

Als Radikalstabilisatoren geeignet sind Inhibitoren und Antioxidantien wie sie in "Methoden der organischen Chemie" (Houben-Weyl), 4. Auflage, Band XIV/1, S. 433ff, Georg Thieme Verlag, Stuttgart 1961, beschrieben sind. Geeignete Stoffklassen sind beispielsweise Phenole wie z.B. 2,6-Di-tert-butyl-4-methylphenol, Kresole, Hydrochinone, Benzylalkohole wie z.B. Benzhydrol, ggf. auch Chinone wie z. B. 2,5-Di-tert.-Butylchinon, ggf. auch aromatische Amine wie Diisopropylamin oder Phenothiazin. Bevorzugte Radikalstabilisatoren sind 2,6-Di-tert.-butyl-4-methylphenol, Phenothiazin und Benzhydrol.

Ferner können ein oder mehrere Katalysatoren eingesetzt werden. Diese katalysieren bevorzugt die Urethanbildung. Dazu sind bevorzugt Amine sowie Metallverbindungen der Metalle Zinn, Zink, Eisen, Bismuth, Molybdän, Kobalt, Calzium, Magnesium und Zirkonium geeignet. Besonders bevorzugt sind Zinnoctoat, Zinkoktoat, Dibutylzinndilaurat, Dimethylzinndicarboxylat, Eisen(III)-acetylacetonat, Eisen(II)-chlorid, Zinkchlorid, Tetraalkylammoniumhydroxide, Alkalihydroxide, Alkalialkoholate, Alkalisalze von langkettigen Fettsäuren mit 10 bis 20 Kohlenstoffatomen und gegebenenfalls seitenständigen OH-Gruppen, Bleioctoat oder tertiäre Amine wie Triethylamin, Tributylamin, Dimethylbenzylamin, Dicyclohexylmethylamin, Dimethylcyclohexylamin, N,N,N',N'-Tetramethyldiaminodiethylether, Bis-(dimethylaminopropyl)harnstoff, N-Methyl- bzw. N-Ethylmorpholin, N,N'-Dimorpholinodiethylether (DMDEE), N-Cyclohexylmorpholin, N,N,N',N'-Tetramethylethylendiamin, N,N,N',N'-Tetramethylbutandiamin, N,N,N',N'-Tetramethyl-hexandiamin-1,6, Pentamethyldiethylentriamin, Dimethylpiperazin, N-Dimethylaminoethylpiperidin, 1,2-Dimethylimidazol, N-Hydroxypropylimidazol, 1-Azabicyclo-(2,2,0)-octan, 1,4-Diazabicyclo-(2,2,2)-octan (Dabco) oder Alkanolaminverbindungen, wie Triethanolamin, Triisopropanolamin, N-Methyl- und N-Ethyldiethanolamin, Dimethylaminoethanol, 2-(N,N-Dimethylaminoethoxy)ethanol oder N-Tris-(dialkylaminoalkyl)hexahydrotriazine, z.B. N,N',N-Tris-(dimethylaminopropyl)-s-hexahydrotriazin 1,4-Diazabicyclo-[2.2.2]-octan, Diazabicyclononan, Diazabicycloundecan, 1,1,3,3-Tetramethylguanidin, 1,3,4,6,7,8-Hexahydro-1-methyl-2H-pyrimido(1,2-a)pyrimidin.

Besonders bevorzugte Katalysatoren sind Dibutylzinndilaurat, Dimethylzinndicarboxylat, Eisen(III)acetylacetonat, 1,4-Diazabicyclo-[2.2.2]-octan, Diazabicyclononan, Diazabicycloundecan, 1,1,3,3-Tetramethylguanidin, 1,3,4,6,7,8-Hexahydro-1-methyl-2H-pyrimido(1,2-a)pyrimidin.

Als weitere Hilfs- und Zusatzstoffe können beispielsweise enthalten sein Lösemittel, Weichmacher, Verlaufsmittel, Benetzungsmittel, Entschäumer oder Haftvermittler, aber auch Polyurethane, thermoplastische Polymere, Oligomere, weitere funktionelle Gruppen, wie z.B. Acetale, Epoxid, Oxetane, Oxazoline, Dioxolane und/oder hydrophile Gruppen, wie z.B. Salze und/oder Polyethylenoxide, aufweisende Verbindungen.

Als Lösemittel werden dabei bevorzugt leichtflüchtige Lösemittel mit guter Verträglichkeit mit den erfindungsgemäßen Formulierungen verwendet, beispielsweise Ethylacetat, Butylacetat, Aceton.

Als Weichmacher werden dabei bevorzugt Flüssigkeiten mit guten Löseeigenschaften, geringer Flüchtigkeit und hoher Siedetemperatur eingesetzt, z.B. können dies sein Diisobutyladipat, Di-n-butyladipat, Dibutylphthalat, nicht-Hydroxy-funktionelle Polyether wie beispielsweise Polyethylenglykoldimethylether mit zahlenmittlerer Molmasse von 250 g/Mol bis 2000 g/Mol oder Polypropylenglykoldimethylether bzw. Mischungen der genannten Verbindungen.

Es kann auch von Vorteil sein, gleichzeitig mehrere Zusatzstoffe eines Typs zu verwenden. Selbstverständlich kann es ebenfalls von Vorteil sein, mehrere Zusatzstoffe mehrerer Typen zu verwenden.

Schichten, Schichtaufbauten und Formkörper erhältlich aus Formulierungen, die die erfindungsgemäßen Photopolymerformulierungen enthalten, weisen ferner typischerweise $\Delta$n-Werte von größer 0.010, bevorzugt größer 0.014, besonders bevorzugt größer 0.017, ganz besonders bevorzugt größer 0.020 auf.

Die erfindungsgemäßen Photopolymerformulierungen eignen sich daher hervorragend zur Herstellung von holographischen Medien und holographischen Photopolymer-Filmen.

Ein weiterer Gegenstand der vorliegenden Erfindung ist daher Verwendung der erfindungsgemäßen Medien zur Aufzeichnung visueller Hologramme oder zur Herstellung von optischen Elementen, Bildern, Darstellungen.

Ebenfalls ein Gegenstand der Erfindung ist daher ein Verfahren zur Belichtung der erfindungsgemäßen Medien, bei dem Schreibmonomere durch aktinische Strahlung selektiv auspolymerisiert werden.

Derartige holographische Medien eignen sich nach entsprechender holographischer Belichtung zur Herstellung von holographischen optischen Elementen, die z.B. die Funktion einer optischen Linse, eines Spiegels, eines Umlenkspiegels, eines Filters, einer Streuscheibe, eines Beugungselements, eines Lichtleiters, eines Lichtlenkers, einer Projektionsscheibe und/oder einer Maske haben.

Zudem können damit auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können, u.a auch in Kombination mit den zuvor dargestellten Produkten.

## <u>Beispiele</u>

Sofern nicht abweichend vermerkt beziehen sich alle Prozentangaben auf Gewichtsprozent.

### Messung der Brechungsindizes der photopolymerisierbaren Monomere

Der Brechungsindex n in Abhängigkeit von der Wellenlänge der Proben wurde aus den Transmissions- und Reflexionsspektren erhalten. Dazu wurden ca. 100 - 300 nm dicke Filme der Proben auf Quarzglasträger aus verdünnter Lösung in Butylacetat aufgeschleudert. Das Transmissions- und Reflexionsspektrum dieses Schichtpaketes wurde mit einem Spektrometer der Firma STEAG ETA-Optik, CD-Measurement System ETA-RT gemessen und danach die Schichtdicke und der spektrale Verlauf von n an die gemessenen Transmissions- und Reflexionsspektren angepasst. Dies geschieht mit der internen Software des Spektrometers und erfordert zusätzlich die Brechungsindexdaten des Quarzglassubstrates, die in einer Blindmessung vorab bestimmt wurden. Der Brechungsindex $n_{Mo}$ bezieht sich auf die Wellenlänge einer Natriumdampflampe von 589 nm und entspricht damit $n_D^{20}$.

### Messung der Brechungsindizes der Matrix, basierend auf einem polymeren Urethan-Netzwerk

Zur Herstellung der Photopolymermatrizes zur Bestimmung des Brechungsindexes $n_{Ma}$ wird die Isocyanatreaktive Komponente b) gegebenenfalls auf 60 °C erhitzt. Dann wird die Isocyanatkomponente a) zugegeben und im Speedmixer (Firma Hauschild) 1 Minute gemischt. Im Folgenden wird eine Lösung der Komponente c) zugegeben und im Speedmixer erneut 1 Minute gemischt. Die Lösung der Komponente c) ist 10 Gewichtsprozent in n-Ethyl-Pyrrolidon. Die entsprechend verwendeten Mengen Lösung fmdet man in Tabelle 1. Die noch flüssige Formulierung wird in der gewünschten Dicke auf Glasplatten aufgerakelt.

Die auf einem polymeren Netzwerk basierende Matrix wurde als ca. 500 $\mu$m bis 1000 $\mu$m dicke Schicht auf einem Glasträger präpariert. An dieser Probe wurde mittels eines Abbe Refraktometers analog zu DIN 51423-2 der Brechungsindex $n_{Ma}$ bei der Wellenlänge der Natriumdampflampe von 589 nm bestimmt und entspricht damit $n_D^{20}$.

### Messung der holographischen Eigenschaften DE und An der holographischen Medien mittels Zweistrahlinterferenz in Reflexionsanordnung

Die wie im Abschnitt "Herstellung der holographischen Medien basierend auf Photopolymerformulierung mit Photoinitiatator zur Bestimmung der Performance Parameter E und $\Delta$n" beschrieben hergestellten Medien wurden

anschließend mittels einer Messanordnung gemäß Figur 1 wie folgt auf ihre holographischen Eigenschaften geprüft:

Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls of 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel ($\alpha$) des Referenzstrahls beträgt 21.8°, der Einfallswinkel ($\beta$) des Signalstrahl beträgt 41.8°. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologtamm). Der Streifenabstand Λ, auch Gitterperiode genannt, im Medium beträgt ~ 225 nm (der Brechungsindex des Mediums zu ~1.504 angenommen).

Figur 1 zeigt den holographischen Versuchsaufbau, mit dem die Beugungseffizienz (DE) der Medien gemessen wurde. Figur 1 zeigt die Geometrie eines HMT bei $\lambda$ = 633 nm (He-Ne Laser): M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha$ = 21.8°, $\beta$ = 41.8° sind die Einfallswinkel der kohärenten Strahlen ausserhalb der Probe (des Mediums) gemessen.

Es wurden auf folgende Weise Hologramme in das Medium geschrieben:

- Beide Shutter (S) sind für die Belichtungszeit t geöffnet.
- Danach wurde bei geschlossenen Shuttem (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega$ = 0° bis $\Omega$ = 20° mit einer Winkelschrittweite von 0.05°. An jedem angefahrenen Winkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$, des geschriebenen Hologramms gemessen und in einem Computer gespeichert. Zusätzlich wurde auch die in die nullte Ordnung transmittierte Intensität gegen der Drehwinkel $\Omega$ aufgezeichnet und in einem Computer gespeichert.

Die maximale Beugungseffizienz (DE = $\eta_{max}$) des Hologramms, also sein Spitzenwert, wurde ermittelt. Eventuell musste dazu die Position des Detektors des abgebeugten Strahls verändert werden, um diesen maximalen Wert zu bestimmen.

Der Brechungsindexkontrast $\Delta n$ und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Das Verfahren wird im folgenden beschrieben:

Für die Braggkurve $\eta(\Omega)$ eines Reflexionshologramms gilt nach Kogelnik:

$$\eta = \frac{1}{1 + \frac{1-(\chi/\Phi)^2}{\sinh^2\left(\sqrt{\Phi^2 - \chi^2}\right)}}$$

mit:

$$\Phi = \frac{\pi \cdot \Delta n \cdot d}{\lambda \cdot \sqrt{\cos(\alpha') \cdot \cos(\alpha' - 2\psi)}}$$

$$\chi = \Delta\theta \cdot \frac{2\pi \cdot \sin(\alpha' - \psi)}{\Lambda \cdot \cos(\alpha' - 2\psi)} \cdot \frac{d}{2}$$

$$\psi = \frac{\beta' - \alpha'}{2}$$

$$\Lambda = \frac{\lambda}{2 \cdot n \cdot \cos(\psi - \alpha')}$$

$$n \cdot \sin(\alpha') = \sin(\alpha), n \cdot \sin(\beta') = \sin(\beta)$$

$$\Delta\theta = -\Delta\Omega \cdot \sqrt{\frac{1 - \sin^2(\alpha)}{n^2 - \sin^2(\alpha)}}$$

$\Phi$ ist die Gitterstärke, $\chi$ ist der Detuning Parameter und $\psi$ der Kippwinkel des Brechungsindexgitters das geschrieben wurde. $\alpha'$ und $\beta'$ entsprechen den Winkeln $\alpha$ und $\beta$ beim Schreiben des Hologramms, aber im Medium gemessen. $\Delta\theta$ ist das Winkeldetuning gemessen im Medium, also die Abweichung vom Winkel $\alpha'$. $\Delta\Omega$ ist das Winkeldetuning gemessen ausserhalb des Mediums, also die Abweichung vom Winkel $\alpha$. n ist der mittlere Brechungsindex des Photopolymers und wurde zu 1.504 gesetzt. $\lambda$ ist die Wellenlänge des Laserlichts im Vakuum.

Die maximale Beugungseffizienz (DE = $\eta_{max}$) ergibt sich dann für $\chi = 0$, also $\Delta\Omega = 0$ zu:

$$DE = \tanh^2(\Phi) = \tanh^2\left(\frac{\pi \cdot \Delta n \cdot d}{\lambda \cdot \sqrt{\cos(\alpha') \cdot \cos(\alpha' - 2\psi)}}\right)$$

Die Messdaten der Beugungseffizienz, die theoretische Braggkurve und die transmittierte Intensität werden wie in Figure 2 gezeigt gegen den zentrierten Drehwinkel $\Omega$-$\alpha$-Shift aufgetragen. Da wegen geometrischem Schrumpf und der Änderung des mittleren Brechungsindexes bei der Photopolymerisation der Winkel bei dem DE gemessen wird von $\alpha$ abweicht wird die x-Achse um diesen Shift zentriert. Der Shift beträgt typischerweise 0° bis 2°.

Da DE bekannt ist wird die Form der theoretischen Braggkurve nach Kogelnik nur noch durch die Dicke d der Photopolymerschicht bestimmt. $\Delta n$ wird über DE für gegebene Dicke d so nachkorrigiert, dass Messung und Theorie von DE immer übereinstimmen. d wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für theoretische Braggkurve und die transmittierte Intensität übereinstimmen.

Da die Richtung in der ein Reflexionshologramm bei der Rekonstruktion mittels eines $\Omega$-Scans mitrotiert, der

Detektor für das abgebeugte Licht aber nur einen endlichen Winkelbereich erfassen kann, wird die Braggkurve von breiten Holgrammen (kleines d) bei einem Ω-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke d zusätzlich herangezogen.

Figur 2 zeigt die Darstellung der Braggkurve η nach Kogelnik (gestrichelte Linie), des gemessenen Beugungswirkungsgrades (ausgefüllte Kreise) und der transmittierten Leistung (schwarz durchgezogene Linie) gegen das Winkeldetuning ΔΩ. Da wegen geometrischem Schrumpf und der Änderung des mittleren Brechungsindexes bei der Photopolymerisation der Winkel bei dem DE gemessen wird von α abweicht wird die x-Achse um diesen Shift zentriert. Der Shift beträgt typischerweise 0° bis 2°.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis E ergibt sich wie folgt aus den Leistungen der zwei den Winkeln α und β zugeordneten Teilstrahlen ($P_\alpha$ = 0.50 mW und $P_\beta$ = 0.67 mW), der Belichtungszeit t und dem Durchmesser der Irisblende (0.4 cm):

$$E\,(\text{mJ/cm}^2) = \frac{2 \cdot \lfloor P_\alpha + P_\beta \rfloor \cdot t\,(\text{s})}{\pi \cdot 0.4^2\ \text{cm}^2}$$

Die Leistungen der Teilstrahlen wurden so angepasst, dass in dem Medium bei den verwendeten Winkeln α und β, die gleiche Leistungsdichte erreicht wird.

Alternative wurde auch ein dem in Abbildung 1 dargestellten Aufbau äquivalenter Test mit einem grünen Laser mit der Emissionswellenlänge λ im Vakuum von 532 nm durchgeführt. Dabei beträgt α = 11.5° und β = 33.5° und $P_\alpha$ = 2.00 mW und $P_\beta$ = 2.00 mW.

**Messung des Plateaumoduls $G_0$ der Photopolymeren mittels eines Oszillationsrheometers im Rahmen der vorliegenden Erfindung**

Zur Herstellung der Photopolymerformulierung zur Bestimmung des Plateaumoduls $G_0$ werden die Komponente B), sowie gegebenenfalls Additive in der isocyanatreaktiven Komponente b) gegebenenfalls bei 60 °C gelöst. Gegebenenfalls wird maximal 10 Minuten im Trockenschrank auf 60 °C erhitzt. Dann wird Isocyanat Komponente a) zugegeben und im Speedmixer 1 Minute gemischt. Im Folgenden wird eine Lösung der Komponente c) in Butylacetat zugegeben und im Speedmixer erneut 1 Minute gemischt. Die Konzentration der Komponente c) in Butylacetat beträgt 10 Gewichtsprozent. Von dieser Lösung wurden die in Tabelle 2 beschriebenen Mengen eingesetzt.

Die noch flüssige Formulierung wird dann in das Platte - Platte Messsystem eines Rheometers (Firma Anton Paar Physica Modell MCR 301 ausgerüstet mit dem Ofenmodel CTD 450 der auf 50°C vorgeheizt war) eingebracht. Dann wird die Aushärtung der Matrix der Photopolymerformulierung über die Zeit unter folgenden Bedingungen gemessen:

- Plattenabstand 250µm.
- Messmodus Oszillation bei einer konstanten Kreisfrequenz $\omega_0$ von 10 rad/s und einer geregelten Deformationsamplitude von 1%.
- Temperatur 50°C, Normalkraftregelung auf 0 Newton eingestellt
- Aufzeichnung des Speichermoduls G' über der Messzeit für mindestens 2 Stunden oder bis ein konstanter Wert $G_{max}$ von G' erreicht wurde.

Anschließend wurde an der Photopolymerformulierung ein Frequenzsweep durchgeführt, um sicherzustellen, dass ein für ein polymeres Netzwerk charakteristischer Plateaumodul $G_0$ erreicht wurde. Folgende Bedingungen wurden gewählt:

- Messmodus Oszillation über einen Kreisfrequenzbereich von 0.5 rad/s < ω < 300 rad/s und einer geregelten Deformationsamplitude von 1 %.
- Temperatur 50°C, Normalkraftregelung auf 0 Newton eingestellt

- Aufzeichnung des Speichermoduls G' über der Kreisfrequenz ω.

Variiert G' um weniger als 30%, bezogen auf den Höchstwert, innerhalb des angegebenen Kreisfrequenzbereich, wird $G_{max}$ als der zu bestimmende Plateaumodul $G_0$ aufgefasst. Beispiele für typische Messkurven finden sich in Figur 3.

Figur 3 zeigt den Verlauf der Aushärtung des Matrixnetzwerkes (links) und Prüfung auf Plateauverhalten (G' unabhängig von ω) (rechts).

Der Plateaumodul $G_0$ kann nach (M. Doi, S.F. Edwards, The Theory of Polymer Dynamics, Oxford Science Publications, 1986) mit dem mittleren Molekulargewicht $M_c$ der zwei Polymerstränge verbrückenden Segmente wie folgt in Beziehung gesetzt werden.

$$G_0 = \frac{\rho \cdot R \cdot T}{M_C}$$

R ist die Avogadro Konstante, T die absolute Temperatur in Kelvin und ρ die Massendichte, die der Einfachheit halber immer zu 1 g/cm$^3$ gesetzt wurde. Ein kleiner Plateaumodul $G_0$ oder ein großes mittleres Molekulargewicht $M_C$ der zwei Polymerstränge verbrückenden Segmente charakterisieren ein Netzwerk mit geringer Vernetzungsdichte.

**Verwendete Isocyanate (Komponenten a)**

Desmodur® XP 2410 ist ein Versuchsprodukt der Bayer MaterialScience AG, Leverkusen, DE, Hexandiisocyanat-basiertes Polyisocyanat, Anteil an Iminooxadiazindion mindestens 30 %, NCO-Gehalt: 23,5 % (Komponente a1)

Desmodur® XP 2580 ist ein Versuchsprodukt der Bayer MaterialScience AG, Leverkusen, DE, aliphatisches Polyisocyanat auf Basis von Hexandiisocyanat, NCO-Gehalt ca. 20 % (Komponente a2)

Desmodur® XP 2599 ist ein Versuchsprodukt der Bayer MaterialScience AG, Leverkusen, DE, Vollallophanat von Hexandiisocyanat auf Acclaim 4200, NCO-Gehalt: 5,6 - 6,4 % (Komponente a3)

<u>Herstellung von Komponente 4a)</u>

Komponente 4a) ist ein experimentelles Produkt der Bayer MaterialScience AG, Leverkusen, DE, Urethan von Hexandiisocyanat und Acclaim 4200, NCO-Gehalt: 18.5%.

In einem Rundkolben wurden 315,0 g Hexamethylendiisocyanat (HDI) unter Rühren und Durchperlen von $N_2$ vorgelegt und 0,016 g Isophthalsäuredichlorid sowie 2 Tropfen (ca. 0,040 g) Dibutylzinndilaurat zugegeben. Die Mischung wurde auf 100 °C erwärmt und über 75 Minuten 478,68 g Acclaim 4200 (Polypropylenoxid der zahlenmittleren Molmasse 4000 g/Mol) zugegeben. Es wurde so lange gerührt, bis ein NCO-Wert von 18,5 % NCO erreicht wurde. Die Reaktion wird dann durch Abkühlen auf Raumtemperatur gestoppt. Dann wird das überschüssige HDI durch Destillation über einen Dünnschichtverdampfer bei 140°C abgetrennt (Rest HDI < 0.1 %). Das Produkt wird als farblose Flüssigkeit erhalten. Es hat teilweise Allophantstrukturen und eine mittlere Funktionalität von ca. 2,6.

**Verwendete isocyanatreaktiven Komponenten (Komponente b)**

<u>Herstellung von Polyol b1:</u>

Polyol b1 ist ein experimentelles Produkt der Bayer MaterialScience AG, Leverkusen, DE, Blockcopolymer von Terathane® 650 und ε-Caprolacton.

In einem 1 L Kolben wurden 0.25 g Zinnoktoat, 172,29 g ε-Caprolacton und 27,46 g eines difunktionellen Polytetrahydrofuranpolyetherpolyols (Equivalentgewicht 325 g/Mol OH) vorgelegt und auf 150 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als viskose Flüssigkeit erhalten.

<u>Herstellung von Polyol b2:</u>

Polyol b2 ist ein experimentelles Produkt der Bayer MaterialScience AG, Leverkusen, DE, Blockcopolymer von Terathane® 1000 und ε-Caprolacton.

In einem 1 L Kolben wurden 0.18 g Zinnoktoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange

auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

Polyol b3 ist ein Polypropylenoxid der zahlenmittleren Molmasse 4000 g/Mol, welches unter dem Handelsnamen Acclaim® 4200 von der Firma Bayer MaterialScience, Leverkusen, DE, vertrieben wird.

Polyol b4 ist ein difunktioneller Mischpolyether aus Ethylen- und Propylenoxid mit gesamt 50 % Ethylenoxidanteil des Equivalentgewichts 984,2 g/Mol

Herstellung von Polyol b5:

In einen mit Rührwerk ausgestatteten 20 1 - Reaktionskessel wurden 3,621 kg Terathane® 1000 eingewogen und 525 mg DMC-Katalysator zugegeben. Dann wurde unter Rühren bei ca. 70 U/min. auf 105 °C erhitzt. Durch dreimaliges Anlegen von Vakuum und Entspannen mit Stickstoff wurde Luft gegen Stickstoff ausgetauscht. Nach Erhöhung der Rührerdrehzahl auf 300 U/min.wurde für 54 Minuten bei laufender Vakuumpumpe und einem Druck von etwa 0,1 bar von unten Stickstoff durch die Mischung geleitet. Danach wurden mittels Stickstoff ein Druck von 0,2 bar eingestellt und 363 g Propylenoxid (PO) zum Start der Polymerisation eingeleitet. Hierbei stieg der Druck auf 2,42 bar. Nach 7 Minuten war der Druck wieder auf 0,34 bar abgefallen und es wurden über einen Zeitraum von 2h 29 min. weitere 11,379 kg PO bei 2,9 bar eindosiert. 47 Minuten nach Ende der PO-Dosierung wurde bei einem Restdruck von 1,9 bar Vakuum angelegt und vollständig entgast. Der Produkt wurde durch Zugabe von 7,5 g Irganox 1076 stabilisiert und als farblose, viskose Flüssigkeit erhalten (OH-Zahl: 27,6 mg KOH/g, Viskosität bei 25 °C: 1498 mPas).

Der Einsatzstoff DMC Katalysator ist ein Doppelmetallcyanid-Katalysator auf Basis Zinkhexacyanocobaltat (III), erhältlich nach dem in EP-A 700 949 beschriebenen Verfahren

**Verwendeter Katalysator (Komponente c)**

Fomrez® UL28: Urethanisierungskatalysator, Dimethylbis[(1-oxoneodecyl)oxy]stannan, Handelsprodukt der Fa. Momentive Performance Chemicals, Wilton, CT, USA (als 10%ige Lösung in N-Ethylpyrrolidon eingesetzt) (Komponente cl).

**Verwendete strahlenhärtende Gruppen (Komponente B)**

Komponente B1): Ethoxylated (3) bisphenol A diacrylate

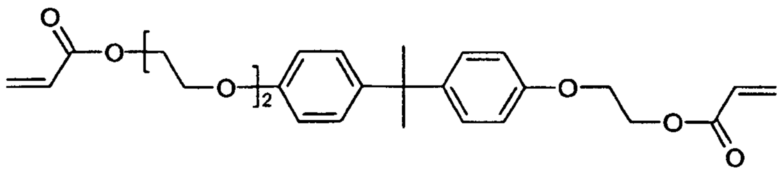

Sartomer Company, 502 Thomas Jones Way Exton, PA 19341 (USA).

Der Brechungsindex $n_D^{20} = n_{Mo}$ beträgt 1.543 (Angaben im Datenblatt des Herstellers).

Komponente B2): Propan-2,2-diylbis[(2,6-dibrom-4,1-phenylen)oxy(2-{[3,3,3-tris(4-chlorphenyl)-propanoyl]-oxy}propan-3,1-diyl)oxyethan-2,1-diyl]-diacrylat.

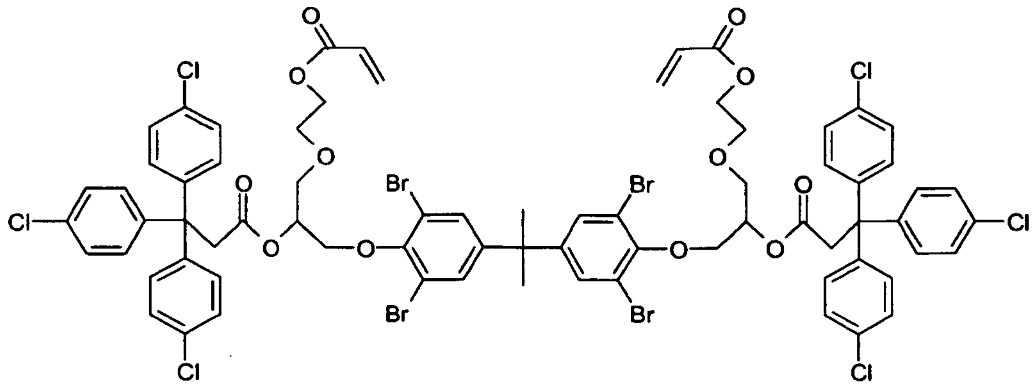

Vorprodukt V1 für Komponente B2)

In einem 6 L Kolben mit Rückfluss-Kühler wurden 215.3 g Tetrabrombisphenol A diglycidylether (D.E.R. 542, Fa. Dow Chemicals, USA) und 1.1 kg Hydroxyethylacrylat in 1.5 L Toluol vorgelegt. Zu dieser Lösung wurden bei Raumtemperatur 1.06 g Bortrifluorid-Diethylether-Komplex getropft und 24 h bei Raumtemperatur nachgerührt. Anschliessend wurde mit 1.3 kg Toluol verdünnt und mit 9g Natriumhydrogencarbonat in 2.5 kg Wasser hydrolysiert. Die abgetrennte organische Phase wurde dreimal mit 2.5 kg Wasser gewaschen und mittels GC auf Hydroxyethylacrylat geprüft. Die org. Phase wurde mit Magnesium-Sulfat getrocknet. Danach wurde das Lösungsmittel bei 5 mbar abdestilliert und abgekühlt.

In einem 6L Kolben mit Rückfluss-Kühler wurden 140.2 g des Vorproduktes V1 in 1.5 kg tert-Butylmethylether vorgelegt und bei Raumtemperatur gelöst. Es wurden 136.8 g 3,3,3-Tris-(4-Chlorphenyl)-propionsäure, 3.67 g Dimethylaminopyrdin und 69.3 g Dicyclohexylcarbodiimid zugeben. Nach kurzer Zeit setzte bei gleichzeitiger Ausfällung eine leicht exotherme Reaktion ein. Es wurde 1 h bei RT nachgerührt. Es wurde filtriert und der Rückstand zweimal mit je 875 mL 0.2 Mol/M wässriger Salzsäure gewaschen. Das Filtrat wurde dann 30 min. mit gesättigte 875 mL NaCl-Lsg. gerührt und anschließend im Scheidetrichter getrennt. Die org. Phase wurde viermal mit 875 mL gesättigter NaCl-Lsg. gewaschen und dann mit Magnesium-Sulfat getrocknet. Der Rückstand wurde mit 0.88g 2,6-Di-tert.-butyl-4-methylphenol (KB) versetzt. Danach wurde das Lösungsmittel bei 5 mbar abdestilliert und abgekühlt. und dreimal mit 2.6 L Isopropanol ausgekocht und abgekühlt. Der erhaltene Rückstand wurde in 1.3 L tert-Butylmethylether aufgenommen, mit Kieselgur versetzt, filtriert und das Lösungsmittel bei 5 mbar abdestilliert und abgekühlt.

Der Brechungsindex $n_{Mo}$ beträgt 1.603.

Komponente B3): Phosphorthioyltris(oxy-4,1-phenyleniminocarbonyloxyethan-2,1-diyl)-triacrylat

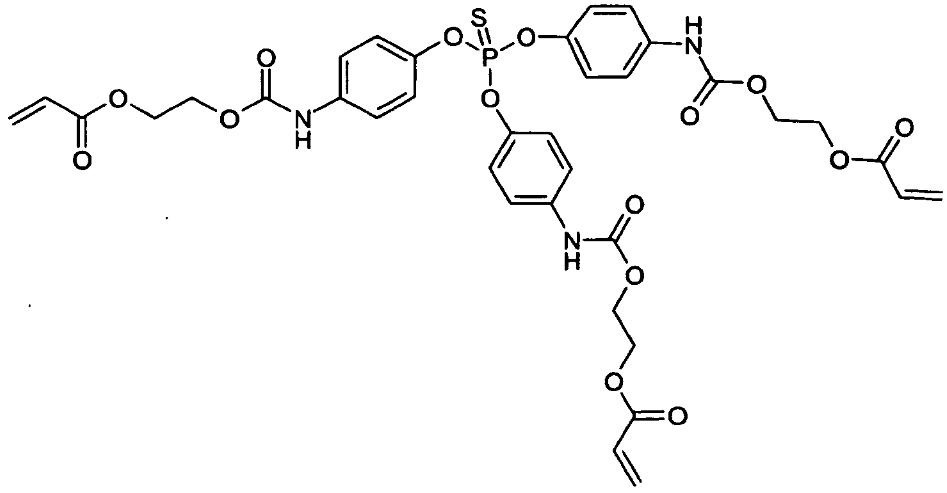

In einem 500 mL Rundkolben werden 0,1 g 2,6-Di-tert.butyl-4-methylphenol, 213,07 g einer 27% igen Lösung von Tris(p-isocyanatophenyl)thiophosphate in Ethylacetat (Desmodur RFE, Produkt der Bayer MaterialScience AG) vorgelegt und auf 60 °C erwärmt. Anschließend werden 42,37 g 2-Hydroxyethylacrylat zugetropft und die Mischung weiter auf 60 °C gehalten, bis der Isocyanatgehalt unter 0,1 % gesunken ist. Danach wird abgekühlt und im Vakuum das Ethylacetat vollständig entfernt. Das Produkt wird als teilkristalliner Feststoff erhalten.

Der Brechungsindex $n_{Mo}$ beträgt 1.579.

Komponente B4): 2-({[3-(Methylsulfanyl)phenyl]carbamoyl}oxy)ethylprop-2-enoat.

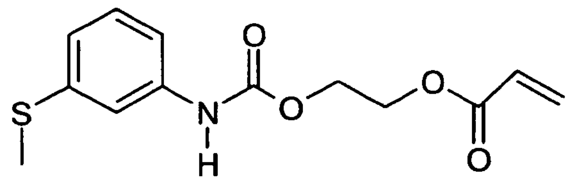

In einem 100 mL Rundkolben wurden 0.02 g 2,6-Di-tert.-butyl-4-methylphenol, 0.01 g Desmorapid Z, 11.7 g 3-(Methylthio)phenylisocyanat vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten.

Der Brechungsindex $n_{Mo}$ beträgt 1.576.

**Verwendete Photoinitiatorsysteme (Komponente C)**

Beschreibung des Systems New Methylen Blue + CGI (Komponente C1))

In einem Becherglas werden im Dunkeln oder unter geeigneter Beleuchtung 0.1g Neu Methylenblau, 1.00 g CGI 909 (Versuchsprodukt der Fa. Ciba Inc., Basel, Schweiz) in 3.50 g n-Ethyl-Pyrrolidon gelöst. Von dieser Lösung werden die entsprechenden Gewichtsprozente (siehe Tabelle 3) zur Erstellung der Beispielmedien verwendet.

Beschreibung des Systems Safranin O + CGI (Komponente C2))

In einem Becherglas werden im Dunkeln oder unter geeigneter Beleuchtung 0.1 g Safranin O, 1.00 g CGI 909 (Versuchsprodukt der Fa. Ciba Inc., Basel, Schweiz) in 3.50 g N-Ethylpyrolidon gelöst. Von dieser Lösung werden die entsprechenden Gewichtsprozente (siehe Tabelle 3) zur Erstellung der Beispielmedien verwendet.

**Folgende dreidimensional vernetzte Polymere als Matrix Komponente A) der Photopolymerformulierung zur Bestimmung des Brechungsindexes $n_{Ma}$ wurden nach dem oben beschriebenen Verfahren hergestellt.**

Tabelle 1 beschreibt die genauen Zusammensetzungen.

| Matrix | Isocyanat Komponente | Anteil (gr) | isocyanatreaktive Komponente | Anteil (gr) | NCO : OH | Katalysator in Lösung | Anteil (gr) | $n_{Ma}$ |
|---|---|---|---|---|---|---|---|---|
| A1 | a1 | 26.8 | b1 | 72.8 | 1.02 : 1 | c1 | 0.4 | 1.485 |
| A2 | a1 | 15.4 | b2 | 84.2 | 1.02 : 1 | c1 | 0.4 | 1.478 |
| A3 | a2 | 2.7 | b4 | 12.3 | 1.02 : 1 | c1 | 0.018 | 1.470 |
| A4 | a2 | 9.6 | b5 | 90.0 | 1.02 : 1 | c1 | 0.4 | 1.460 |
| A5 | a3 | 26.6 | b3 | 73.0 | 1.02 : 1 | c1 | 0.4 | 1.455 |
| A6 | a3 | 42.1 | b4 | 57.5 | 1.02 : 1 | c1 | 0.4 | 1.465 |

Tabelle 1: Matrixkomponenten A) zur Bestimmung des Brechungsindexes $n_{Ma}$ bei 589 nm. NCO:OH bezeichnet das Äquivalentverhältnis der funktionellen Gruppen in den Komponenten a) und b) in der jeweiligen Komponente A)

**Herstellung der Photopolymerformulierung ohne Photoinitiatator zur Bestimmung des Plateaumoduls $G_0$.**

Tabelle 2 listet die untersuchten Beispiele der Photopolymerformulierungen zur Bestimmung des Plateaumoduls $G_0$, die nicht ausschließenden Charakter haben in ihrer Zusammensetzung auf. Diese Photopolymerformulierungen wurden entsprechend der Vorschrift, die im Abschnitt zur Messung des Plateaumoduls $G_0$ der Photopolymeren mittels eines Oszillationsrheometers beschrieben wurde hergestellt.

| Photopolymer Formulierung ohne Photoinitiator | Isocyanat Komponente | Anteil (gr) | isocyanatreaktive Komponente | Anteil (gr) | NCO : OH | Strahelnhärtende Komponente | Anteil (Gew. %) | Karalysator in Lösung | Anteil (gr) |
|---|---|---|---|---|---|---|---|---|---|
| F1 | a1 | 2.432 | b1 | 6.566 | 1.02 : 1 | B3 | 12.5 | c1 | 0.0107 |
| F2 | a1 | 2.027 | b1 | 5.472 | 1.02 : 1 | B3 | 25.0 | c1 | 0.0106 |
| F3 | a1 | 1.406 | b2 | 7.593 | 1.02 : 1 | B3 | 12.5 | c1 | 0.0104 |
| F4 | a1 | 1.172 | b2 | 6.328 | 1.02 : 1 | B2 | 25.0 | c1 | 0.0104 |
| F5 | a1 | 1.172 | b2 | 6.328 | 1.02 : 1 | B3 | 25.0 | c1 | 0.0104 |
| F6 | a1 | 1.063 | b2 | 6.437 | 1 : 0.90 | B3 | 25.0 | c1 | 0.0100 |
| F7 | a3 | 3.202 | b4 | 4.295 | 1.02 : 1 | B3 | 25.0 | c1 | 0.0328 |
| F8 | a3 | 0.725 | b5 | 6.773 | 1.02 : 1 | B3 | 25.0 | c1 | 0.0331 |
| F9 | a3 | 5.510 | b3 | 1.987 | 1.02 : 1 | B3 | 25.0 | c1 | 0.0318 |
| F10 | a3 | 1.722 | b3 | 4.775 | 1.02 : 1 | B3 | 35.0 | c1 | 0.0300 |
| F11 | a4 | 4.223 | b3 | 2.310 | 1.02 : 1 | B3 | 22.5 | c1 | 0.0664 |
| F12 | a1 | 1.117 | b2 | 6.031 | 1.02 : 1 | B4 | 25.0 | c1 | 0.0080 |
| F13 | a3 | 1.960 | b3 | 5.187 | 1.02 : 1 | B4 | 25.0 | c1 | 0.0180 |
| F14 | a1 | 1.117 | b2 | 6.023 | 1.02 : 1 | B1 | 25.0 | c1 | 0.0111 |
| F17 | a2 | 1.361 | b4 | 6.161 | 1.02 : 1 | B3 | 20.0 | c1 | 0.0201 |

Tabelle 2: **Photopolymerformulierungen die auf ihren Plateaumodul $G_0$ und ihre Vernetzungsdichte $1/M_C$ untersucht wurden.**

**Herstellung der holographischen Medien basierend auf Photopolymerformulierung mit Photoinitiatator zur Bestimmung der Performance Parameter E und Δn.**

Es wurden aus den Photopolymerformulierungen holographische Medien hergestellt (siehe Tabelle 3), in denen sich das Photopolymer als Schicht zwischen Glasscheiben von je 1 mm Dicke hergestellt. Diese Art der holographischen Medien ist besonders geeignet, um deren Performance nach der im Abschnitt Messung der holographischen Eigenschaften DE und Δn der holographischen Medien mittels Zweistrahlinterferenz in Reflektionsanordnung beschriebenen Verfahren zu bestimmen und ist daher nicht begrenzend gemeint im Sinne der formulierten Ansprüche auf die holographischen Medien solange die verwendeten Photopolymerformulierung den beanspruchten Eigenschaften hinsichtlich Plateaumodul $G_0$ und / oder $Q = M_c / M_{Mo}$ genügen.

**Beispielhafte Herstellung der holographischen Medien**

Zur Herstellung der holografischen Medien werden im Dunkeln die Komponente B), die Komponente C) (die bereits in der Komponente B) vorgelöst sein kann) sowie gegebenenfalls die Additive in der Isocyanat reaktiven Komponente b) gegebenenfalls bei 60 °C gelöst, dann werden Glasperlen der Größe 20 µm (z. B. der Fa. Whitehouse Scientific Ltd, Waverton, Chester, CH3 7PB, United Kingdom) zugegeben und gründlich gemischt (Speedmixer). Gegebenenfalls wird maximal 10 Minuten im Trockenschrank auf 60 °C erhitzt. Dann wird die Isocyanat Komponente a) zugegeben und wieder im Speedmixer 1 Minute gemischt. Im Folgenden wird eine Lösung der Komponente c) zugegeben und im Speedmixer erneut 1 Minute gemischt. Die erhaltene Mischung wird unter Rühren bei < 1 mbar maximal 30 Sekunden entgast, dann wird sie auf Glasplatten von 50 x 75 mm verteilt und diese je mit einer weiteren Glasplatte abgedeckt. Die Härtung der PU-Formulierung erfolgt unter 15 kg Gewichten über mehrere Stunden (gewöhnlich über

Nacht). Teilweise werden die Medien in lichtdichter Verpackung noch 2 Stunden bei 60 °C nachgehärtet. Da unterschiedliche Formulierungen mit unterschiedlicher Ausgangsviskosität und unterschiedlicher Härtungsgeschwindigkeit der Matrix zu nicht immer gleichen Schichtdicken d der Photopolymerschicht führen, wird d anhand der Charakteristika der geschriebenen Hologramme für jede Probe separat ermittelt.

| holographisches Medium | Isocyanat Komponente | Anteil (gr) | isocyanatreaktive Komponente | Anteil (gr) | NCO : OH | Strahlenhärtbare Komponente | Anteil (Gew. %) | Photinitiator | Anteil (Gew. %) | Katalysator in Lösung | Anteil (gr) | Entspricht Photopolymerformuleirung ohne Photoinitiator |
|---|---|---|---|---|---|---|---|---|---|---|---|---|
| M1 | a1 | 20.5 | b1 | 55.7 | 1.02 : 1 | B3 | 12.5 | C1 | 4.6 | c1 | 0.0060 | F1 |
| M2 | a1 | 18.8 | b1 | 51.3 | 1.02 : 1 | B3 | 25.0 | C1 | 4.6 | c1 | 0.0040 | F2 |
| M3 | a1 | 12.9 | b2 | 69.7 | 1.02 : 1 | B3 | 12.5 | C1 | 4.6 | c1 | 0.0090 | F3 |
| M4 | a1 | 22.2 | b2 | 72.9 | 1.02 : 1 | B2 | 25.0 | C1 | 4.6 | c1 | 0.0060 | F4 |
| M5 | a1 | 11.0 | b2 | 59.3 | 1.02 : 1 | B3 | 25.0 | C1 | 4.6 | c1 | 0.0060 | F5 |
| M6 | a1 | 10.0 | b2 | 60.3 | 1 : 0.90 | B3 | 25.0 | C1 | 4.6 | c1 | 0.0060 | F6 |
| M7 | a3 | 29.9 | b4 | 40.1 | 1.02 : 1 | B3 | 25.0 | C1 | 4.6 | c1 | 0.0446 | F7 |
| M8 | a2 | 6.8 | b5 | 63.3 | 1.02 : 1 | B3 | 25.0 | C1 | 4.6 | c1 | 0.0363 | F8 |
| M9 | a3 | 18.6 | b3 | 51.4 | 1.02 : 1 | B3 | 25.0 | C1 | 4.6 | c1 | 0.0315 | F9 |
| M10 | a3 | 15.9 | b3 | 44.2 | 1.02 : 1 | B3 | 35.0 | C1 | 4.6 | c1 | 0.0360 | F10 |
| M11 | a4 | 38.5 | b3 | 31.6 | 1.02 : 1 | B3 | 22.5 | C1 | 4.6 | c1 | 0.0275 | F11 |
| M12 | a1 | 11.0 | b2 | 59.3 | 1.02 : 1 | B4 | 25.0 | C1<br>C2 | 4.6<br>4.6 | c1 | 0.0090 | F12 |
| M13 | a3 | 19.2 | b3 | 50.9 | 1.02 : 1 | B4 | 25.0 | C2 | 4.6 | c1 | 0.0300 | F13 |
| M14 | a1 | 11.0 | b2 | 59.3 | 1.02 : 1 | B1 | 25.0 | C1 | 4.6 | c1 | 0.0060 | F14 |
| M17 | a2 | 13.6 | b4 | 61.5 | 1.02 : 1 | B3 | 20.0 | C1 | 4.6 | c1 | 0.0336 | F17 |

Tabelle 3: Holographische Medien die auf ihre Performance Δn und E geprüft wurden.

**Ergebnisse aus $G_0$, $M_C$, $M_{Mo}$ und Δn kombiniert.**

Folgende Messwerte für $G_0$ (MPa), $M_c$ (g/mol), $Q = M_C/M_{Mo}$, und Δn bei der Dosis E (mJ/cm$^2$) wurden erhalten und sind in Tabelle 4 dargestellt:

| Beispiel Typ | Holographische Medieum | $\Delta n$ | E | NCO : OH | Entsprechende Photopolymerformulierung ohne Photoinitiator | $G_0$ | $M_C$ | $Q = M_C/M_{Mo}$ | $n_{Mo}$-$n_{Ma}$ | Entsprechende Matrix |
|---|---|---|---|---|---|---|---|---|---|---|
| Vergleich | M1 | 0.0062 | 8.1 | 1.02 : 1 | F1 | 2.25 | 1194 | 1.47 | 0.094 | A1 |
| Vergleich | M2 | 0.0059 | 15.9 | 1.02 : 1 | F2 | 1.75 | 1535 | 1.89 | 0.094 | A1 |
| Vergleich | M3 | 0.0070 | 15.9 | 1.02 : 1 | F3 | 1.60 | 1678 | 2.06 | 0.103 | A2 |
| Vergleich | M4 | 0.0059 | 36.9 | 1.02 : 1 | F4 | 1.35 | 1989 | 1.21 | 0.127 | A2 |
| Erfin. Ge. | M5 | 0.0101 | 7.8 | 1.02 : 1 | F5 | 0.80 | 3357 | 4.13 | 0.103 | A2 |
| Erfin. Ge. | M6 | 0.0113 | 9.1 | 1 : 0.90 | F6 | 0.70 | 3836 | 4.72 | 0.103 | A2 |
| Erfin. Ge. | M7 | 0.0130 | 8.9 | 1.02 : 1 | F7 | 0.70 | 3836 | 4.72 | 0.114 | A6 |
| Erfin. Ge. | M12 | 0.0118<br>0.0110* | 18.2<br>127.3* | 1.02 : 1 | F12 | 0.53 | 5067 | 18.03 | 0.098 | A2 |
| Erfin. Ge. | M17 | 0.0130 | 9.1 | 1.02 : 1 | F17 | 0.43 | 6231 | 7.66 | 0.109 | A3 |
| Erfin. Ge. | M14 | 0.0103 | 18.3 | 1.02 : 1 | F14 | 0.36 | 7460 | 15.94 | 0.065 | A2 |
| Erfin. Ge. | M8 | 0.0147 | 9.1 | 1.02 : 1 | F8 | 0.30 | 8951 | 11.01 | | |
| Erfin. Ge. | M9 | 0.0170 | 9.1 | 1.02 : 1 | F9 | 0.25 | 10742 | 13.21 | 0.124 | A5 |
| Erfin. Ge. | M10 | 0.0208 | 4.5 | 1.02 : 1 | F10 | 0.24 | 11189 | 13.76 | 0.124 | A5 |
| Erfin. Ge. | M13 | 0.011* | 63.7* | 1.02 : 1 | F13 | 0.10 | 26854 | 95.57 | 0.121 | A5 |
| Erfin. Ge. | M11 | 0.0171 | 4.7 | 1.02 : 1 | F11 | 0.05 | 53708 | 66.06 | | |

Tabelle 4: **Bewertung ausgewählter Beispiele. Die mit * gekennzeichneten Werte wurden statt mit $\lambda = 633$ nm mit $\lambda = 532$ nm gemessen.**

Die gefundenen Werte für $\Delta n$ der holographischen Medien zeigen überraschenderweise, dass Photopolymerformulierungen deren Plateaumodul $G_0$ kleiner als **1.0** MPa ist, bzw. deren äquivalentes mittleres Molekulargewicht $M_c$ der zwei Polymerstränge verbrückenden Segmente größer als **2685** g/mol ist, besonders bevorzugt deren Verhältnis $M_C/M_{Mo}$ größer als **3.30** ist für die Verwendung in holographischen Medien sehr gut geeignet sind, da $\Delta n$ Werte größer als **0.010** erreicht werden.

Darüberhinaus zeigen sie dass dieses Designkriterium für Photopolymerformulierungen gleiche oder sogar höhere Signifikanz besitzt als der Brechungsindexunterschied zwischen Matrix und photopolymerisierbaren Monomeren **(siehe z.B. M14 im Vergleich zu M7 bzw. zu M4),** bzw. dass dieses Designkriterium bestehende Photopolymerformulierungen, bei denen dieser Indexunterschied fest ist in ihrer Performance weiter verbessert **(siehe z.B. M6 im Vergleich zu M5 bzw. zu M3).**

**Patentansprüche**

1. Photopolymerformulierungen umfassend dreidimensional vernetzte organische Polymere A) oder deren Vorstufen als Matrix sowie Verbindungen B), die unter Einwirkung aktinischer Strahlung mit ethylenisch ungesättigten Verbindungen unter Polymerisation reagierende Gruppen (strahlenhärtende Gruppen) aufweisen und in dieser Matrix gelöst oder verteilt vorliegen sowie C) wenigstens einen Photoinitiator, **gekennzeichnet durch** eine Netzwerkdichte des organischen Polymers ausgedrückt **durch** das mittlere Molekulargewicht $M_c$ der zwei Polymerstränge verbrükkenden Segmente von mindestens 2685 g/mol.

2. Photopolymerformulierungen gemäß Anspruch 1, **dadurch gekennzeichnet, dass** $M_c$ im Bereich von 7500 bis

55000 g/mol liegt.

3. Photopolymerformulierungen gemäß Anspruch 1 oder 2, **dadurch gekennzeichnet, dass** das Verhältnis Q des Molekulargewichts $M_c$ zum zahlenmittleren Molekulargewicht $M_{Mo}$ der Verbindungen B) größer als 3.30 ist.

4. Photopolymerformulierungen gemäß einem der Ansprüche 1 bis 3, **dadurch gekennzeichnet, dass** das Verhältnis Q größer als 10,00 ist.

5. Photopolymerformulierungen gemäß einem der Ansprüche 1 bis 4, **dadurch gekennzeichnet, dass** die dreidimensional vernetzten organischen Polymere Urethangruppen aufweisen.

6. Photopolymerformulierungen gemäß einem der Ansprüche 1 bis 5, **dadurch gekennzeichnet, dass** die dreidimensional vernetzten organischen Polymere der Komponente A) aufgebaut sind aus einer Isocyanatkomponente a) und einer Isocyanat-reaktiven Komponente b).

7. Photopolymerformulierungen gemäß einem der Ansprüche 1 bis 5, **dadurch gekennzeichnet, dass** die Komponente A) eine Isocyanatkomponente a) und eine Isocyanat-reaktiven Komponente b) umfasst.

8. Photopolymerformulierungen gemäß Anspruch 6 oder 7, **dadurch gekennzeichnet, dass** die Komponente a) Polyisocyanate basierend auf HDI mit Isocyanurat- und/oder Iminooxadiazindionstrukturen oder Präpolymere mit NCO-Funktionalitäten von 2 bis 5 mit Allophanat- und/oder Urethanstrukturen auf Basis von HDI und/oder TMDI und Polyether-, Polyester- und/oder Polycarbonatpolyolen umfasst.

9. Photopolymerformulierungen gemäß einem der Ansprüche 6 bis 8, **dadurch gekennzeichnet, dass** die Komponente b) Polypropylenoxide, Polyethylenoxide und/oder deren Kombinationen in Form von statistischen oder Blockcopolymeren und/oder Blockcopolymere der vorstehend genannten Art, die zusätzlich Tetrahydrofuran, Butylenoxid oder ε-Caprolacton als Monomereinheiten enthalten umfasst, wobei die OH-Funktionalitäten von 1,5 bis 6 und die zahlenmittleren Molekulargewichte 200 bis 18000 g/Mol betragen.

10. Photopolymerformulierungen gemäß einem der Ansprüche 1 bis 9, **dadurch gekennzeichnet, dass** die in Komponente B) eingesetzten Verbindungen einen Brechungsindex $n_D^{20}$ von größer 1.54 aufweisen.

11. Photopolymerformulierungen gemäß einem der Ansprüche 1 bis 10, **dadurch gekennzeichnet, dass** die in Komponente B) eingesetzten Verbindungen als strahlenhärtende Gruppen Acrylat- und/oder Methacrylatgruppen aufweisen.

12. Medien geeignet zur Aufzeichnung von visuellen Hologrammen erhältlich aus Photopolymerformulierungen gemäß einem der Ansprüche 1 bis 11.

13. Verwendung von Medien gemäß Anspruch 12, zur Aufzeichnung visueller Hologramme oder zur Herstellung von optischen Elementen, Bildern, Darstellungen.

14. Verfahren zur Belichtung von Medien gemäß Anspruch 12, bei dem Schreibmonomere durch aktinische Strahlung selektiv auspolymerisiert werden.

## Claims

1. Photopolymer formulations comprising three-dimensionally crosslinked organic polymers A) or the precursors thereof as a matrix and compounds B) which have groups redacting with ethylenically unsaturated compounds with polymerization under the action of actinic radiation (radiation-curing groups) and are present in solution or dispersion in this matrix and C) at least one Photoinitiator, **characterized by** a network density of the organic polymer, expressed by the average molecular weight $M_c$ of the segments bridging two polymer strands, of at least 2685 g/mol.

2. Photopolymer formulations according to Claim 1, **characterized in that** $M_C$ is in the range from 7500 to 55 000 g/mol.

3. Photopolymer formulations according to Claim 1 or 2, **characterized in that** the ratio Q of the molecular weight $M_c$ to the number average molecular weight $M_{Mo}$ of the compounds B) is greater than 3.30.

4. Photopolymer formulations according to any of Claims 1 to 3, **characterized in that** the ratio Q is greater than 10.00,

5. Photopolymer formulations according to any of Claims 1 to 4, **characterized in that** the three-dimensionally crosslinked organic polymers have urethane groups.

6. Photopolymer formulations according to any of Claims 1 to 5, **characterized in that** the three dimensionally crosslinked organic polymers of component A) arc composed of an isocyanate component a) and an isocyanate-reactive component b).

7. Photopolymer formulations according to any of Claims 1 to 5, **characterized in that** the component A) comprises an isocyanate component a) and an isocyanate-reactive component b).

8. Photopolymer formulations according to Claim 6 or 7, **characterized in that** component a) comprises polyisocyanates based on HDI with isocyanurate and/or iminooxadiazinedione structures or prepolymers having NCO functionalities of 2 to 5 with allophanate and/or urethane structures based on HDI and/or TMDI and polyether polyols, polyester polyols and/or polycarbonate polyols.

9. Photopolymer formulations according to any of Claims 6 to 8, **characterized in that** component b) comprises polypropylene oxides, polyethylene oxides and/or combinations thereof in the form of random or block copolymers and/or block copolymers of the abovementioned type which additionally contain tetrahydrofuran, butylene oxide or ε-caprolactone as monomer units, the OH functionalities being 1.5 to 6 and the number average molecular weights being 200 to 18 000 g/mol

10. Photopolymer formulations according to any of Claims 1 to 9, **characterized in that** the compounds used in component B) have a refractive index $n_D^{20}$ of greater than 1.54.

11. Photopolymer formulations according to any of Claims 1 to 10, **characterized in that** the compounds used in component B) have acrylate and/or methacrylate groups as radiation-curing groups.

12. Media suitable for recording visual holograms, obtainable from photopolymer formulations according to any of Claims 1 to 11.

13. Use of media according to Claim 12 for according visual holograms or for producing optical elements, images, representations.

14. Method for exposing media according to Claim 12, in which writhing monomers are selectively polymerized by actinic radiation.

## Revendications

1. Formulations de photopolymères comprenant des polymères organiques réticulés de manière tridimensionnelle A) ou leurs précurseurs comme matrice ainsi que des composés B) qui présentent des groupes réagissait avec des composés éthyléniquement insaturées en polymérisant sous l'effet d'un rayonnement actinique (groupes durcissant sous l'effet de rayons) et qui se trouvent sous forme dissoute ou répartie dans cette matrice ainsi que C) au moins un photo-initiateur, **caractérisées par** une densité de réseau du polymère organique, exprimée par le poids moléculaire moyen $M_c$ des segments formant un pont entre deux brins polymères d'au moins 2685 g/mole.

2. Formulations de photopolymères selon la revendication 1, **caractérisées en ce que** $M_c$ se situe dans la plage de 7500 à 55 000 g/mole.

3. Formulations de photopolymères selon la revendication 1 ou 2, **caractérisées en ce que** le rapport Q du poids moléculaire $M_c$ au poids moléculaire numérique moyen $M_{Mo}$ des composés B) est supérieur à 3,30.

4. Formulations de photopolymères selon l'une quelconque des revendications 1 à 3, **caractérisées en ce que** le rapport Q est supérieur à 10,00.

5. Formulations de photopolymères selon l'une quelconque des revendications 1 à 4, **caractérisées en ce que** les

polymères organiques réticulés de manière tridimensionnelle présentent des groupes uréthane.

6. Formulations de photopolymères selon l'une quelconque des revendications 1 à 5, **caractérisées en ce que** les polymères organiques réticulés de manière tridimensionnelle du composant A) sont formés à partir d'un composant isocyanate a) et d'un composant réactif avec isocyanate b).

7. Formulations de photopolymères selon l'une quelconque des revendications 1 à 5, **caractérisées en ce que** le composant A) comprend un composant isocyanate a) et un composant réactif avec isocyanate b) .

8. Formulations de photopolymères selon la revendication 6 ou 7, **caractérisées en ce que** le composant a) comprend des polyisocyanates à base de HDI avec des structures isocyanurate et/ou iminooxadiazinedione ou des prépolymères présentant des fonctionnalités NCO- de 2 à 5 avec des structures allophanate et/ou uréthanne à base de HDI et/ou de TMDI et des polyétherpolyols, polyesterpolyols et/ou polycarbonatepolyols.

9. Formulations de photopolymères selon l'une quelconque des revendications 6 à 8, **caractérisées en ce que** le composant b) comprend des poly (oxydes de propylène), des poly(oxydes d'éthylène) et/ou leurs combinaisons sous forme de copolymères statistiques ou à blocs et/ou des copolymères à blocs du type susmentionné qui contiennent en outre du tétrahydrofuranne, de l'oxyde de butylène ou de l'ε-caprolactone comme unités monomères, les fonctionnalités OH valant 1,5 à 6 et les poids moléculaires numériques moyens valant 200 à 18 000 g/mole.

10. Formulations de photopolymères selon l'une quelconque des revendications 1 à 9, **caractérisées en ce que** les composés utilisés dans le composant B) présentent un indice de réfraction $n_D^{20}$ supérieur à 1,54.

11. Formulations de photopolymères selon l'une quelconque des revendication 1 à 10, **caractérisées en ce que** les composés utilisés dans le composant B) présentent, comme groupes durcissant sous l'effet de rayons, des groupes acrylate et/ou méthacrylate.

12. Agents appropriés pour enregistrer des hologrammes visuels pouvant être obtenus à partir de formulations de photopolymères selon l'une quelconque des revendications 1 à 11.

13. utilisation d'agents selon la revendication 12 pour enregistrer des hologrammes visuels ou pour la fabrication d'éléments, d'écrans, de représentations optiques.

14. Procédé pour éclairer des agents selon la revendication 12, des monomères d'enregistrement étant polymérisées sélectivement par un rayonnement actinique.

**Figur 1:**

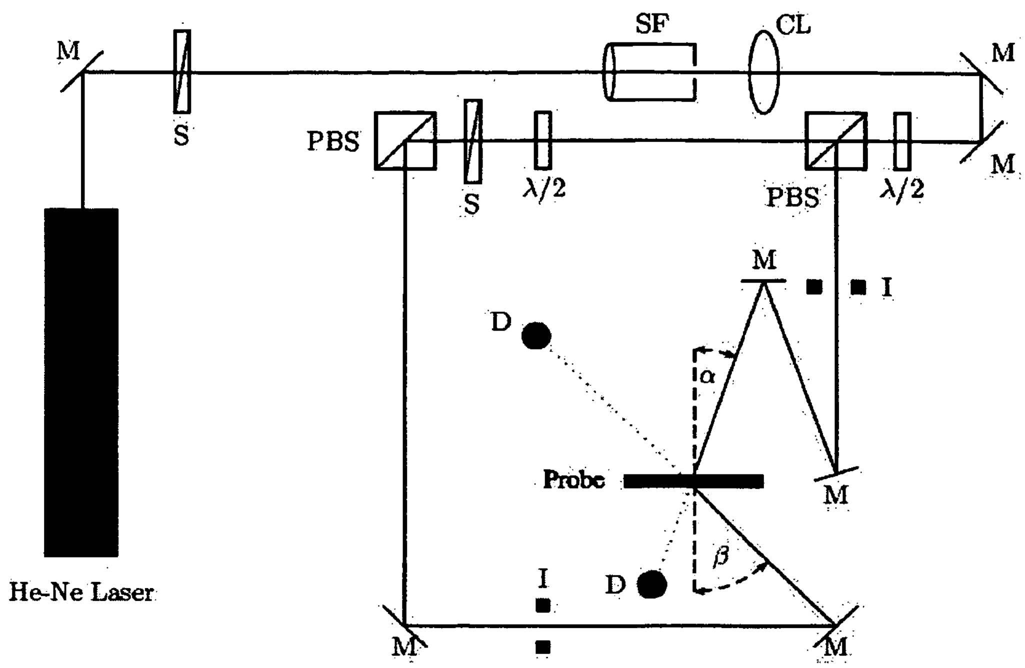


**Figur 2:**

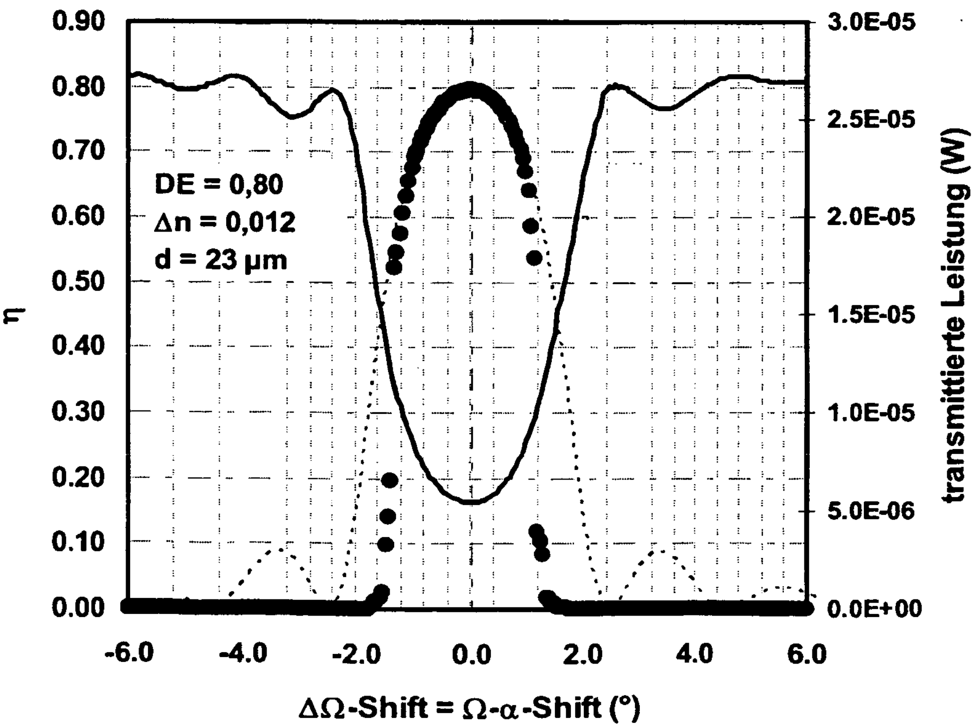

**Figur 3:**

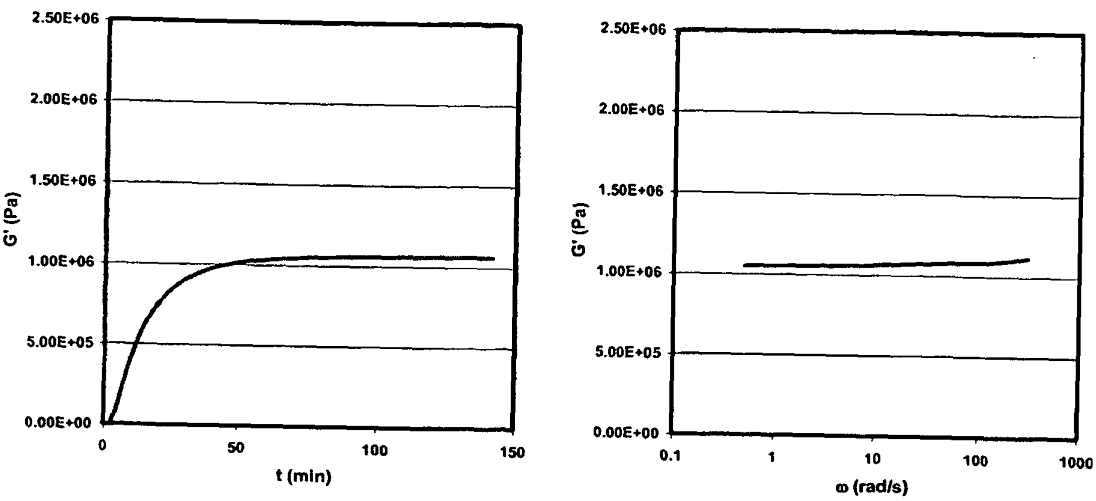
2.50E+06
2.00E+06
1.50E+06
1.00E+06
5.00E+05
0.00E+00
G' (Pa)
0
50
100
150
t (min)
2.50E+06
2.00E+06
1.50E+06
1.00E+06
5.00E+05
0.00E+00
G' (Pa)
0.1
1
10
100
1000
ω (rad/s)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4959284 A, Dupont
- EP 352774 A1, Dupont
- US 020070077498 A
- US 6103454 A
- US 5916987 A
- US 6780546 B
- US 6794471 B
- EP 2008002464 W
- US 6939648 B
- EP 0223587 A
- EP 700949 A


### In der Beschreibung aufgeführte Nicht-Patentliteratur


- **O'Neill et al.** *Applied Optics,* 2002, vol. 41 (5), 845ff
- **Luo et al.** *Optics Express,* 2005, vol. 13 (8), 3123
- *Springer Series in Optical Sciences,* 2000, vol. 76, 209-228
- *Polymer,* 2005, vol. 46, 4735-4742
- Hariharan Optical Holography, Principles, Techniques and Applications. Cambridge University Press, 1991, 44
- Roche Lexikon Medizin. Urban & Fischer Verlag, 1999
- **M. Doi ; S.F. Edwards.** The Theory of Polymer Dynamics. Oxford Science Publications, 1986
- Römpp Lexikon Chemie. Georg-Thieme-Verlag, 1998, 465-466
- Chemistry & Technology of UV & EB Formulations For Coatings, Inks & Paints. SITA Technology, 1991, vol. 2, 37-56
- **Cunningham et al.** *RadTech'98 North America UV/EB Conference Proceedings,* 19. April 1998
- **Kutal et al.** *Macromolecules,* 1991, vol. 24, 6872
- **Yamaguchi et al.** *Macromolecules,* 2000, vol. 33, 1152
- **Neckers et al.** *Macromolecules,* 2000, vol. 33, 7761
- **Dektar et al.** *J. Org. Chem.,* 1990, vol. 55, 639
- *J. Org. Chem.,* 1991, vol. 56, 1838
- **Crivello et al.** *Macromolecules,* 2000, vol. 33, 825
- **Gu et al.** *Am. Chem. Soc. Polymer Preprints,* 2000, vol. 41 (2), 1266
- **Hua et al.** *Macromolecules,* 2001, vol. 34, 2488-2494
- **Houben-Weyl.** Methoden der organischen Chemie. Georg Thieme Verlag, 1961, vol. XIV/1, 433ff
- **H. Kogelnik.** *The Bell System Technical Journal,* November 1969, vol. 48 (9), 2909-2947